(19)

Europäisches
Patentamt

European
Patent Office

Office européen
des brevets

(11) **EP 3 433 612 B1**

(12) **EUROPEAN PATENT SPECIFICATION**

(45) Date of publication and mention
of the grant of the patent:
**09.10.2024 Bulletin 2024/41**

(21) Application number: **17712979.8**

(22) Date of filing: **22.03.2017**

(51) International Patent Classification (IPC):
**G01N 33/50** *(2006.01)*      **G01N 33/68** *(2006.01)*

(52) Cooperative Patent Classification (CPC):
**G01N 33/505; A61P 3/02; A61P 3/10;**
**G01N 33/6893;** G01N 2800/042

(86) International application number:
**PCT/EP2017/056859**

(87) International publication number:
**WO 2017/162759 (28.09.2017 Gazette 2017/39)**

(54) **METHODS AND KITS OF ASSESSING STATUS, RISK OR PROGNOSIS OF TYPE 1 DIABETES**

VERFAHREN UND KITS ZUR BEURTEILUNG DES STATUS, DES RISIKOS ODER DER PROGNOSE
VON TYP-1-DIABETES

PROCÉDÉS ET KITS POUR ÉVALUER LE STATUT, LE RISQUE OU LE PRONOSTIC DU DIABÈTE
DE TYPE 1

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **22.03.2016 EP 16305319**

(43) Date of publication of application:
**30.01.2019 Bulletin 2019/05**

(73) Proprietors:
• **INSERM (Institut National de la Santé**
**et de la Recherche Médicale)**
**75013 Paris (FR)**
• **Université Paris Cité**
**75006 Paris (FR)**
• **Centre National de la Recherche Scientifique**
**(CNRS)**
**75016 Paris (FR)**

(72) Inventors:
• **LEHUEN-MONTEIRO, Agnès**
**75014 Paris (FR)**
• **NEL, Isabelle**
**75014 Paris (FR)**
• **DA SILVA, Jennifer**
**75014 Paris (FR)**

• **BEAUDOIN, Lucie**
**75014 Paris (FR)**

(74) Representative: **Inserm Transfert**
**PariSanté Campus**
**10 rue d'Oradour-sur-Glane**
**75015 Paris (FR)**

(56) References cited:
**US-A1- 2013 316 375**

• **J. KIS ET AL: "Reduced CD4+ subset and Th1 bias**
**of the human iNKT cells in Type 1 diabetes**
**mellitus", JOURNAL OF LEUKOCYTE BIOLOGY,**
**vol. 81, no. 3, 1 March 2006 (2006-03-01), US,**
**pages 654 - 662, XP055277262, ISSN: 0741-5400,**
**DOI: 10.1189/jlb.1106654**
• **ROBERT Z. HARMS ET AL: "Altered CD161bright**
**CD8+ Mucosal Associated Invariant T (MAIT)-Like**
**Cell Dynamics and Increased Differentiation**
**States among Juvenile Type 1 Diabetics", PLOS**
**ONE, vol. 10, no. 1, 27 January 2015 (2015-01-27),**
**pages e0117335, XP055276891, DOI:**
**10.1371/journal.pone.0117335**
• **ISABELLE MAGALHAES ET AL: "iNKT and MAIT**
**Cell Alterations in Diabetes", FRONTIERS IN**
**IMMUNOLOGY, vol. 6, 2 July 2015 (2015-07-02),**
**XP055277266, DOI: 10.3389/fimmu.2015.00341**

**EP 3 433 612 B1**

**Description**

**FIELD:**

The disclosure relates to methods of assessing status, risk or prognosis of type 1 diabetes.

**BACKGROUND:**

Type 1 diabetes (TID) is an autoimmune disease that results from the selective destruction of insulin-producing β-cells in pancreatic islets. The diagnosis of TID is commonly preceded by a long prodromal period which includes seroconversion to islet autoantibody positivity and subtle metabolic disturbances. Thus there is still a need for improved methods of assessing status, risk or prognosis of type 1 diabetes. The incidence of TID among children and adolescents has increased markedly in the Western countries during the recent decades and is presently increasing at a faster rate than ever before. This suggests an important role of environment and gene-environment interactions in TID pathogenesis. It is also hypothesized that gut microbiota may affect its incidence via the modulation of the host innate immune system. Indeed, studies disclose an impact of the T1D on the MAIT dynamic and the frequency of iNKT and MAIT (Harms R.Z et al, Plos One 2015; Magalhaes I et al, Frontiers in Immunology 2015). In particular, the literature describes a decreased diversity in the intestinal microbiota in at risk children with seroconversions who progressed to T1D compared to those who did not progress during the study observation period (Kostic et al, Cell Host & Microbes 2015). Because this observation takes place in the time window between the first seroconversion and much before the T1D diagnosis, it suggests that an event linked to the intestinal microbiota could contribute to the progression of islet autoimmunity towards clinical T1D. Moreover, this decrease of intestinal microbiota diversity seems to be associated with an increased intestinal permeability in at risk children who have at least two autoantibodies (Bosi E et al, Diabetologia 2006 and Graham S et al, Gut 2004). Recently published data also show modification of glycolipids in the stools of at risk children (Kostic et al, Cell Host & Microbes 2015).

**SUMMARY OF THE INVENTION:**

The present invention is defined by the claims. In particular, the present invention relates to a method of identifying whether a subject has or at risk of having type 1 diabetes comprising i) quantifying the population of MAIT cells in the blood sample obtained from the subject, ii) comparing the quantification value determined at step i) with a predetermined reference value and iii) concluding that the subject has or is at risk of having type 1 diabetes when the quantification value at step i) is lower than the predetermined reference value. The following detailed description, figures and example do not fall under the scope of the present invention and are present for understanding and illustration purposes only.

**DETAILED DESCRIPTION:**

The disclosure relates to a method of assessing status, risk or prognosis of type 1 diabetes in a subject comprising i) quantifying at least one population of innate-like T-cells in a blood sample obtained from the subject, ii) comparing the quantification value determined at step i) with a predetermined reference value and iii) detecting differential in the quantification value determined at step i) and the predetermined reference value is indicative of the status, risk or prognosis of type 1 diabetes.

In particular, the subject is child. In particular the child is 3 to 5 years old.

In particular, the subject is an adult. In particular, the adult is more than 18 years old.

In particular, the method of the disclosure is suitable for identifying whether a subject has or is at risk of having type 1 diabetes (i.e. status or risk) in particular in children. In particular, the method of the disclosure is suitable for determining the outcome of type 1 diabetes (i.e. prognosis) in particular in adults.

In particular, the population of innate-like T-cells is a population of MAIT cells. As used herein, the term "MAIT cells" or "Mucosal- Associated Invariant T cells" refers to a population of T cells that display an invariant TCR alpha chain comprising $V\alpha7.2$-Joc33, a CDR3 of constant length, and a limited number of $V\beta$ segments (see, e.g., Lantz and Bendelac. 1994. J. Exp Med. 180:1097-106; Tilloy et al., J. Exp. Med., 1999, 1907-1921; Treiner et al. (2003) Nature 422:164-169). MAIT cells are restricted by the non-classical MHC class I molecule MRI.

In particular, the population of innate-like T-cells is a population of iNKT cells. As used herein, the term "iNKT cells" or "invariant NKT cells" refers to a major subset of NKT cells, also called type 1 NKT cells expressing an invariant natural T cell receptor (TCR) composed of V[alpha] 14-J[alpha] 18 chains in mice (V[alpha]24-J[alpha] 18 in humans). Upon TCR stimulation with a ligand, such as alpha-galactosylceramide, iNKT cells rapidly produce a wide range of cytokines including IL-4, IFN- [gamma], IL-12, and GM-CSF. It should be further noted that iNKT cells comprise two main subsets (or subpopulations), namely CD4+ and CD4-cells, which in humans have distinct cytokine secretion profiles. This rapid and potent response to a ligand enables iNKT cells to enhance or regulate the activity of various immune cells in innate and acquired immunity.

In particular, the population of innate-like T-cells is characterized by the presence or absence of at least one marker of activation, proliferation, exhaustion or cytotoxicity. In particular, the population of innate-like T-cells is characterized by the presence or absence of the expression of at least one cytokine. In particular, the population of innate-like T-cells of the disclosure is characterized by the presence or absence of at least one marker selected from the group consisting of CCR6, CD56, CD25, CD69, CD161, CD27, PD1, TIM3, KLRG1, BTLA, BCL2, Ki67, CD127, GzB, IFNg, IL2, TNFa, IL4, IL10 and IL17.

As used herein the term "CCR6" has its general meaning in the art and refers to chemokine (C-C motif) receptor 6 (Gene ID: 1235). CCR6 is also known as BN-1; DCR2; DRY6; CCR-6; CD196; CKRL3; GPR29; CKR-L3; CMKBR6; GPRCY4; STRL22; CC-CKR-6; and C-C CKR-6.

As used herein the term "CD56" has its general meaning in the art and refers to the neural cell adhesion molecule 1 (Gene ID: 4684). CD56 is also known as MSK39 and NCAM.

As used herein the term "CD161" has its general meaning in the art and refers to the killer cell lectin like receptor B1 (Gene ID: 3820). CD161 is also known as KLRB1, NKR; CD161; CLEC5B; NKR-P1; NKRP1A; NKR-P1A; hNKR-P1A.

As used herein the term "CD27" has its general meaning in the art and refers to the CD27 molecule (Gene ID:939). CD27 is also known as S152, S152. LPFS2, T14, TNFRSF7, and Tp55.

As used herein the term "CD25" has its general meaning in the art and refers to the interleukin 2 receptor subunit alpha (Gene ID: 3559). CD25 is also known as IL2RA, p55; IL2R; IMD41; TCGFR; and IDDM10.

As used herein the term "CD69" has its general meaning in the art and refers to the CD69 molecule (Gene ID: 969). CD69 is also known as AIM, BL-AC/P26, CLEC2C, EA1, GP32/28, and MLR-3.

As used herein the term "PD1" has its general meaning in the art and refers to the programmed cell death 1 (Gene ID: 5133). PD1 is also known as PDCD1, CD279, PD-1, SLEB2, hPD-1, hPD-1, and hSLE1.

As used herein the term "TIM3" has its general meaning in the art and refers to the hepatitis A virus cellular receptor 2 (Gene ID: 84868). TIM3 is also known as HAVCR2, CD366, HAVcr-2, KIM-3, TIMD-3, TIMD3, and Tim-3.

As used herein the term "KLRG1" has its general meaning in the art and refers to the killer cell lectin like receptor G1 (Gene ID: 10219). KLRG1 is also known as 2F1, CLEC15A, MAFA, MAFA-2F1, MAFA-L, and MAFA-LIKE.

As used herein the term "BTLA" has its general meaning in the art and refers to the B and T lymphocyte associated (Gene ID: 151888). BTLA is also known as CD272.

As used herein the term "BCL2" has its general meaning in the art and refers to the B-cell CLL/lymphoma 2 (Gene ID: 596). BCL2 is also known as Bcl-2 and PPP1R50.

As used herein the term "Ki67" has its general meaning in the art and refers to the marker of proliferation Ki-67 (Gene ID: 4288). Ki67 is also known as MKI67, KIA, MIB-, MIB-1, and PPP1R105.

As used herein the term "CD127" has its general meaning in the art and refers to the interleukin 7 receptor (Gene ID: 3575). CD127 is also known as CDW127, IL-7R-alphaA, ILRA, and IL7R.

As used herein the term "GzB" has its general meaning in the art and refers to the granzyme B (Gene ID: 3002). GZB is also known as CCPI, CGL-1, CGL1, CSP-B, CSPB, CTLA1, CTSGL1, HLP, and SECT.

As used herein the term "IFNg" has its general meaning in the art and refers to the interferon, gamma (Gene ID: 3458). IFNg is also known as IFG and IFI.

As used herein the term "IL2" has its general meaning in the art and refers to the interleukin 2 (Gene ID: 3558). IL2 is also known as IL-2, TCGF and lymphokine.

As used herein the term "TNFa" has its general meaning in the art and refers to the tumor necrosis factor (Gene ID: 7124). TNFa is also known as DIF-alpha, TNFA, TNFSF2, TNLG1F, and TNF.

As used herein the term "IL4" has its general meaning in the art and refers to the interleukin 4 (Gene ID: 3565). IL4 is also known as BCGF-1, BCGF1, BSF-1, BSF1, and IL-4.

As used herein the term "IL10" has its general meaning in the art and refers to the interleukin 10 (Gene ID: 3586). IL10 is also known as CSIF, GVHDS, IL-10A, TGIF, and IL10.

As used herein the term "IL17" has its general meaning in the art and refers to the interleukin 17A (Gene ID: 3605). IL17 is also known as CTLA-8, CTLA8, IL-17, IL-17A, and IL 17 A.

In particular, the method of the disclosure comprises quantifying at least one population of MAIT cells expressing 1, 2, 3, 4, 5, or 6 markers selected from the group consisting of CCR6, CD56, CD25, CD69, PD1, TIM3, CD27, KLRG1, BTLA, BCL2, Ki67, CD127, GzB, IFNg, IL2, TNFa, IL4, IL10 and IL17.

In particular, the method of the disclosure comprises quantifying at least one population selected from the group consisting of MAIT BTLA+, MAIT CCR6+, MAIT CD25+, MAIT CD56+, MAIT CD69+, MAIT CD127+, MAIT PD1+, MAIT CD27-, MAIT KLRG1+, MAIT TIM3+, iNKT, iNKT BTLA+, iNKT CCR6+, iNKT CD25+, iNKT CD56+, iNKT CD69+, iNKT CD127+, iNKT CD161+, iNKT PD1+, iNKT CD27-, iNKT KLRG1+, and iNKT TIM3+.

In particular, the method of the disclosure comprises quantifying at least one population selected from the group consisting of MAIT, MAIT CCR6+, MAIT CD25+, MAIT CD56+, MAIT PD1+, iNKT CCR6+, iNKT CD25+ and iNKT PD1+

More particularly, the method of the disclosure comprises quantifying at least one population selected from the group consisting of MAIT, MAIT CCR6+, MAIT CD25+, MAIT CD56+ and MAIT PD1 +.

In particular, the method of the disclosure comprises quantifying at least one population selected from the group consisting of MAIT BCL2+, MAIT Ki67+, MAIT GzB+, MAIT IFNg+, MAIT IL2+, MAIT IL4+, MAIT IL10+, MAIT IL17+, MAIT TNFa+, iNKT BCL2+, iNKT Ki67+, iNKT GzB+, iNKT IFNg+, iNKT IL2+, iNKT IL4+, iNKT IL10+, iNKT IL17+, and iNKT TNFa+.

In particular, the method of the disclosure comprises quantifying at least one population selected from the group consisting of MAIT CCR6+ CD56+, MAIT CCR6- CD56-, MAIT CCR6+ CD25+, MAIT CCR6+ CD25-, MAIT CCR6+ PD1-, MAIT CD25+ CD56-, MAIT CD25+ CCR6-, MAIT CD25+ PD1+, MAIT CD25+ PD1-, MAIT CD25- PD1-, MAIT CD56+ PD1-, MAIT CD56-PD1+, MAIT CD56- PD1-, MAIT CCR6- PD1+, MAIT CD25+ CD56-PD1+, MAIT CD25+ CD56- PD1-, MAIT CCR6+ CD25+ PD1+, MAIT CCR6+ CD25+ PD1-, MAIT CCR6+ CD25- PD1-, MAIT CCR6- CD25+ PD1-, MAIT CCR6+ CD25+ CD56-, MAIT CCR6- CD25+ CD56-, MAIT CCR6+ CD56+ PD1-, MAIT CCR6+ CD25+ CD56- PD1+, MAIT CCR6+ CD25+ CD56- PD1-, MAIT CCR6- CD25+ CD56- PD1-, iNKT CCR6+CD161-, iNKT CD25+PD1+, iNKT CD25-CD161+, iNKT CD69+CD161+, iNKT CD69+CD161-, iNKT CD69-PD1+, iNKT CD161+PD1-, iNKT CCR6-CD25+CD161+, iNKT CD25-CD161+PD1-, iNKT CCR6+CD25-CD161-PD1+, iNKT CCR6-CD25+CD161+PD1+, and iNKT CCR6-CD25+CD161+PD1-.

In particular, the method of the disclosure comprises quantifying at least one population selected from the group consisting of MAIT CD25+ PD1+, MAIT CD25+ PD1-, MAIT CD25+ CCR6-, MAIT CCR6+ CD56+, MAIT CCR6- CD56-, MAIT CD25+ CD56- PD1+, MAIT CCR6- CD25+ PD1-, iNKT CCR6-CD25+CD161+, MAIT CCR6+ CD25+ CD56-, MAIT CCR6+ CD25+ PD1-, MAIT CCR6+ CD25+ PD1+, MAIT CCR6- CD25+ CD56-, MAIT CD25+ CD56- PD1-, , MAIT CCR6+ CD25+ CD56- PD1+, MAIT CCR6+ CD25+ CD56-PD1-, MAIT CCR6- CD25+ CD56- PD1-, iNKT CCR6-CD25+CD161+PD1-, and iNKT CCR6-CD25+CD161+PD1+.

More particularly, the method of the disclosure comprises quantifying at least one population selected from the group consisting of MAIT CCR6+ CD56+, MAIT CCR6- CD56-, MAIT CD25+ PD1+, and MAIT CD25+ CD56- PD1+.

In particular, the method of the disclosure comprises quantifying at least one population selected from the group consisting of MAIT IFNg+ IL4+, MAIT IFNg- IL4-, MAIT IFNg+ GrB-, MAIT IFNg- GrB+, MAIT IFNg+ IL17-, MAIT IFNg-IL17+, MAIT IFNg- IL17-, MAIT IL4+ GrB-, MAIT IL4- GrB+, MAIT IL17+ GrB+, MAIT IL17- GrB-, MAIT GrB-IFNg+ IL4+, MAIT GrB- IFNg+ IL4-, and MAIT GrB+ IFNg- IL4-.

In particular, when the subject is a child, the method of the disclosure comprises:

- i) quantifying the population of MAIT cells in the blood sample obtained from the subject, ii) comparing the quantification value determined at step i) with a predetermined reference value and iii) concluding that the subject has or is at risk of having type 1 diabetes when the quantification value at step i) is lower than the predetermined reference value, or
- i) quantifying the population of MAIT CCR6+ cells in the blood sample obtained from the subject, ii) comparing the quantification value determined at step i) with a predetermined reference value and iii) concluding that the subject has or is at risk of having type 1 diabetes when the quantification value at step i) is lower than the predetermined reference value, or
- i) quantifying the population of MAIT CD25+ cells in the blood sample obtained from the subject, ii) comparing the quantification value determined at step i) with a predetermined reference value and iii) concluding that the subject has or is at risk of having type 1 diabetes when the quantification value at step i) is higher than the predetermined reference value
- i) quantifying the population of MAIT CD69+ cells in the blood sample obtained from the subject, ii) comparing the quantification value determined at step i) with a predetermined reference value and iii) concluding that the subject has or is at risk of having type 1 diabetes when the quantification value at step i) is higher than the predetermined reference value, or
- i) quantifying the population of MAIT CD56+ cells in the blood sample obtained from the subject, ii) comparing the quantification value determined at step i) with a predetermined reference value and iii) concluding that the subject has or is at risk of having type 1 diabetes when the quantification value at step i) is lower than the predetermined reference value, or
- i) quantifying the population of MAIT PD1+ cells in the blood sample obtained from the subject, ii) comparing the quantification value determined at step i) with a predetermined reference value and iii) concluding that the subject has or is at risk of having type 1 diabetes when the quantification value at step i) is higher than the predetermined reference value, or
- i) quantifying the population of MAIT TIM3+ cells in the blood sample obtained from the subject, ii) comparing the quantification value determined at step i) with a predetermined reference value and iii) concluding that the subject has or is at risk of having type 1 diabetes when the quantification value at step i) is higher than the predetermined reference value, or

- i) quantifying the population of MAIT KLRG1+ cells in the blood sample obtained from the subject, ii) comparing the quantification value determined at step i) with a predetermined reference value and iii) concluding that the subject has or is at risk of having type 1 diabetes when the quantification value at step i) is higher than the predetermined reference value, or

- i) quantifying the population of MAIT BTLA+ cells in the blood sample obtained from the subject, ii) comparing the quantification value determined at step i) with a predetermined reference value and iii) concluding that the subject has or is at risk of having type 1 diabetes when the quantification value at step i) is higher than the predetermined reference value, or

- i) quantifying the population of MAIT BCL2+ cells in the blood sample obtained from the subject, ii) comparing the quantification value determined at step i) with a predetermined reference value and iii) concluding that the subject has or is at risk of having type 1 diabetes when the quantification value at step i) is lower than the predetermined reference value, or

- i) quantifying the population of MAIT Ki67+ cells in the blood sample obtained from the subject, ii) comparing the quantification value determined at step i) with a predetermined reference value and iii) concluding that the subject has or is at risk of having type 1 diabetes when the quantification value at step i) is higher than the predetermined reference value, or

- i) quantifying the population of MAIT GzB+ cells in the blood sample obtained from the subject, ii) comparing the quantification value determined at step i) with a predetermined reference value and iii) concluding that the subject has or is at risk of having type 1 diabetes when the quantification value at step i) is higher than the predetermined reference value, or

- i) quantifying the population of MAIT IFNg+ cells in the blood sample obtained from the subject, ii) comparing the quantification value determined at step i) with a predetermined reference value and iii) concluding that the subject has or is at risk of having type 1 diabetes when the quantification value at step i) is lower than the predetermined reference value, or

- i) quantifying the population of MAIT TNFa+ cells in the blood sample obtained from the subject, ii) comparing the quantification value determined at step i) with a predetermined reference value and iii) concluding that the subject has or is at risk of having type 1 diabetes when the quantification value at step i) is higher than the predetermined reference value, or

- i) quantifying the population of MAIT IL4+ cells in the blood sample obtained from the subject, ii) comparing the quantification value determined at step i) with a predetermined reference value and iii) concluding that the subject has or is at risk of having type 1 diabetes when the quantification value at step i) is higher than the predetermined reference value, or

- i) quantifying the population of MAIT IL10+ cells in the blood sample obtained from the subject, ii) comparing the quantification value determined at step i) with a predetermined reference value and iii) concluding that the subject has or is at risk of having type 1 diabetes when the quantification value at step i) is higher than the predetermined reference value, or

- i) quantifying the population of MAIT IL17+ cells in the blood sample obtained from the subject, ii) comparing the quantification value determined at step i) with a predetermined reference value and iii) concluding that the subject has or is at risk of having type 1 diabetes when the quantification value at step i) is higher than the predetermined reference value.

In particular, when the subject is an adult, the method of the disclosure comprises:

- i) quantifying the population of MAIT cells in the blood sample obtained from the subject, ii) comparing the quantification value determined at step i) with a predetermined reference value and iii) concluding that the subject has or is at risk of having type 1 diabetes when the quantification value at step i) is lower than the predetermined reference value, or

- i) quantifying the population of MAIT CD25+ cells in the blood sample obtained from the subject, ii) comparing the quantification value determined at step i) with a predetermined reference value and iii) concluding that the subject has or is at risk of having type 1 diabetes when the quantification value at step i) is higher than the predetermined reference value

- i) quantifying the population of MAIT CD69+ cells in the blood sample obtained from the subject, ii) comparing the quantification value determined at step i) with a predetermined reference value and iii) concluding that the subject has or is at risk of having type 1 diabetes when the quantification value at step i) is higher than the predetermined reference value, or

- i) quantifying the population of MAIT CD56+ cells in the blood sample obtained from the subject, ii) comparing the quantification value determined at step i) with a predetermined reference value and iii) concluding that the subject has or is at risk of having type 1 diabetes when the quantification value at step i) is higher than the predetermined reference value, or

- i) quantifying the population of MAIT Ki67+ cells in the blood sample obtained from the subject, ii) comparing the quantification value determined at step i) with a predetermined reference value and iii) concluding that the subject has or is at risk of having type 1 diabetes when the quantification value at step i) is higher than the predetermined reference value, or

- i) quantifying the population of MAIT IFNg+ cells in the blood sample obtained from the subject, ii) comparing the quantification value determined at step i) with a predetermined reference value and iii) concluding that the subject has or is at risk of having type 1 diabetes when the quantification value at step i) is lower than the predetermined reference value, or

- i) quantifying the population of MAIT TNFa+ cells in the blood sample obtained from the subject, ii) comparing the quantification value determined at step i) with a predetermined reference value and iii) concluding that the subject has or is at risk of having type 1 diabetes when the quantification value at step i) is lower than the predetermined reference value.

In particular, when the subject is a child or an adult, the method of the disclosure comprises:

- i) quantifying the population of iNKT cells in the blood sample obtained from the subject, ii) comparing the quantification value determined at step i) with a predetermined reference value and iii) concluding that the subject has or is at risk of having type 1 diabetes when the quantification value at step i) is lower than the predetermined reference value, or

- i) quantifying the population of iNKT CD25+ cells in the blood sample obtained from the subject, ii) comparing the quantification value determined at step i) with a predetermined reference value and iii) concluding that the subject has or is at risk of having type 1 diabetes when the quantification value at step i) is higher than the predetermined reference value, or

- i) quantifying the population of iNKT CD161+ cells in the blood sample obtained from the subjecte, ii) comparing the quantification value determined at step i) with a predetermined reference value and iii) concluding that the subject has or is at risk of having type 1 diabetes when the quantification value at step i) is lower than the predetermined reference value, or

- i) quantifying the population of iNKT CD69+ cells in the blood sample obtained from the subject, ii) comparing the quantification value determined at step i) with a predetermined reference value and iii) concluding that the subject has or is at risk of having type 1 diabetes when the quantification value at step i) is lower than the predetermined reference value, or

- i) quantifying the population of iNKT PD1+ cells in the blood sample obtained from the subject, ii) comparing the quantification value determined at step i) with a predetermined reference value and iii) concluding that the subject has or is at risk of having type 1 diabetes when the quantification value at step i) is higher than the predetermined reference value, or

- i) quantifying the population of iNKT KLRG1+ cells in the blood sample obtained from the subject, ii) comparing the quantification value determined at step i) with a predetermined reference value and iii) concluding that the subject has or is at risk of having type 1 diabetes when the quantification value at step i) is higher than the predetermined reference value, or

- i) quantifying the population of iNKT BCL2+ cells in the blood sample obtained from the subject, ii) comparing the quantification value determined at step i) with a predetermined reference value and iii) concluding that the subject has or is at risk of having type 1 diabetes when the quantification value at step i) is lower than the predetermined reference value,

- i) quantifying the population of iNKT CD127+ cells in the blood sample obtained from the subject, ii) comparing the quantification value determined at step i) with a predetermined reference value and iii) concluding that the subject has or is at risk of having type 1 diabetes when the quantification value at step i) is lower than the predetermined reference value,

- i) quantifying the population of iNKT IFNg+ cells in the blood sample obtained from the subject, ii) comparing the quantification value determined at step i) with a predetermined reference value and iii) concluding that the subject has or is at risk of having type 1 diabetes when the quantification value at step i) is lower than the predetermined reference value,

- i) quantifying the population of iNKT IL2+ cells in the blood sample obtained from the subject, ii) comparing the quantification value determined at step i) with a predetermined reference value and iii) concluding that the subject has or is at risk of having type 1 diabetes when the quantification value at step i) is lower than the predetermined reference value,

- i) quantifying the population of iNKT TNFa+ cells in the blood sample obtained from the subject, ii) comparing the quantification value determined at step i) with a predetermined reference value and iii) concluding that the subject has or is at risk of having type 1 diabetes when the quantification value at step i) is lower than the predetermined reference value.

In particular, at least 2, 3, 4, 5, 6, 7, 8, 9, or 10 population of cells are quantified.

In particular, the method of the disclosure comprises quantifying the population of MAIT CCR6+, MAIT CD56+, MAIT CCR6+ CD56+, MAIT CCR6- CD56-, MAIT CD25+ PD1+ and MAIT CD25+ CD56- PD1+.

In particular, the blood sample is a PBMC sample. As used herein, the term "PBMC" or "peripheral blood mononuclear cells" or "unfractionated PBMC" refers to whole PBMC, i.e. to a population of white blood cells having a round nucleus, which has not been enriched for a given sub-population. Typically, these cells can be extracted from whole blood using Ficoll, a hydrophilic polysaccharide that separates layers of blood, with the PBMC forming a cell ring under a layer of plasma. Additionally, PBMC can be extracted from whole blood using a hypotonic lysis which will preferentially lyse red blood cells. Such procedures are known to the expert in the art.

In particular, the quantification is absolute or relative. In particular, when the quantification is relative, it consists in determining the frequency of the population in the general population of T cells (i.e. characterized by the expression of CD3) or the frequency of the population in the population of MAIT or iNKT cells.

The quantification of the population of innate-like T-cells is determined by any method well known in the art and typically involves flow cytometry methods. As used herein, the term "flow cytometric method" refers to a technique for counting cells of interest, by suspending them in a stream of fluid and passing them through an electronic detection apparatus. Flow cytometric methods allow simultaneous multiparametric analysis of the physical and/or chemical parameters of up to thousands of events per second, such as fluorescent parameters. Modern flow cytometric instruments usually have multiple lasers and fluorescence detectors. A common variation of flow cytometric techniques is to physically sort particles based on their properties, so as to purify or detect populations of interest, using "fluorescence-activated cell sorting". As used herein, "fluorescence-activated cell sorting" (FACS) refers to a flow cytometric method for sorting a heterogeneous mixture of cells from a biological sample into two or more containers, one cell at a time, based upon the specific light scattering and fluorescent characteristics of each cell and provides fast, objective and quantitative recording of fluorescent signals from individual cells as well as physical separation of cells of particular interest. Accordingly, FACS can be used with the methods described herein to isolate and detect the population of cells of the disclosure. For example, fluorescence activated cell sorting (FACS) may be therefore used. It involves using a flow cytometer capable of simultaneous excitation and detection of multiple fluorophores, such as a BD Biosciences FACSCanto™ flow cytometer, used substantially according to the manufacturer's instructions. The cytometric systems may include a cytometric sample fluidic subsystem, as described below. In addition, the cytometric systems include a cytometer fluidically coupled to the cytometric sample fluidic subsystem. Systems of the present disclosure may include a number of additional components, such as data output devices, e.g., monitors, printers, and/or speakers, softwares (e.g. (Flowjo, Kaluza.... ), data input devices, e.g., interface ports, a mouse, a keyboard, etc., fluid handling components, power sources, etc. More particularly, the blood sample is contacted with a panel of antibodies specific for the specific market of the population of cells of the interest. As used herein, the term "antibody" refers to an intact immunoglobulin or to a monoclonal or polyclonal antigen-binding fragment with the Fc (crystallizable fragment) region or FcRn binding fragment of the Fc region. Antigen-binding fragments may be produced by recombinant DNA techniques or by enzymatic or chemical cleavage of intact antibodies. "Antigen-binding fragments" include, inter alia, Fab, Fab', F(ab')2, Fv, dAb, and complementarity determining region (CDR) fragments, single -chain antibodies (scFv), single domain antibodies, chimeric antibodies, diabodies and polypeptides that contain at least a portion of an immunoglobulin that is sufficient to confer specific antigen binding to the polypeptide. The terms Fab, Fc, pFc', F(ab') 2 and Fv are employed with standard immunological meanings (Roitt, I. (1991) Essential Immunology, 7th Ed., (Blackwell Scientific Publications, Oxford)]. Such antibodies or antigen-binding fragments are available commercially from vendors such as R&D Systems, BD Biosciences, e- Biosciences, Biolegend, Proimmune and Miltenyi, or can be raised against these cell-surface markers by methods known to those skilled in the art. In particular, an agent that specifically bind to a cell-surface marker, such as an antibody or antigen-binding fragment, is labelled with a tag to facilitate the isolation and detection of population of cells of the interest. As used herein, the terms "label" or "tag" refer to a composition capable of producing a detectable signal indicative of the presence of a target, such as, the presence of a specific cell-surface marker in a biological sample. Suitable labels include fluorescent molecules, radioisotopes, nucleotide chromophores, enzymes, substrates, chemiluminescent moieties, magnetic particles, bioluminescent moieties, and the like. As such, a label is any composition detectable by spectroscopic, photochemical, biochemical, immunochemical, electrical, optical or chemical means needed for the methods to isolate and detect immune cells or iNKT cell populations and MAIT cell populations. Fluorescent labels or tags for labeling the agents such as antibodies for use in the methods of disclosure comprise Hydroxycoumarin, Succinimidyl ester, Aminocoumarin, Succinimidyl ester, Methoxycoumarin, Succinimidyl ester, Cascade Blue, Hydrazide, Pacific Blue, Maleimide, Pacific Orange, Lucifer yellow, NBD, NBD-X, R-Phycoerythrin (PE), a PE-Cy5 conjugate (Cychrome, R670, Tri-Color, Quantum Red), a PE-Cy7 conjugate, Red 613, PE-Texas Red, PerCP, PerCPeFluor 710, PE-CF594, Peridinin chlorphyll protein, TruRed (PerCP-Cy5.5 conjugate), FluorX, Fluoresceinisothyocyanate (FITC), BODIPY-FL, TRITC, X-Rhodamine (XRITC), Lissamine Rhodamine B, Texas Red, Allophycocyanin (APC), an APC-Cy7 conjugate, Alexa Fluor 350, Alexa Fluor 405, Alexa Fluor 430, Alexa Fluor 488, Alexa Fluor 500, Alexa Fluor 514, Alexa Fluor 532, Alexa Fluor 546, Alexa Fluor 555, Alexa Fluor 568, Alexa Fluor 594, Alexa Fluor 610, Alexa Fluor 633, Alexa Fluor 647, Alexa Fluor 660, Alexa Fluor 680, Alexa Fluor 700, Alexa Fluor 750, Alexa Fluor 790, Cy2, Cy3, Cy3B, Cy3.5, Cy5, Cy5.5, Cy7, BV 785, BV711, BV421, BV605, BV510 or BV650. The aforementioned assays may involve the binding of the antibodies to a solid support. The solid surface could be a microtitration plate coated with the antibodies. Alternatively, the solid surfaces may be beads, such as activated beads, magnetically responsive beads. Beads may be made of different materials, comprising glass, plastic, polystyrene, and acrylic. In addition, the beads are preferably fluorescently labelled. In particular, fluorescent beads are those contained in TruCount(TM) tubes, available from Becton Dickinson Biosciences, (San Jose, California). In particular, PBMC were stained for detection of cytokines production after stimulation in RPMI medium supplemented with 10% fetal bovine serum with PMA and ionomycin at 25ng/mL and 1$\mu$g/mL, respectively, in the presence of brefeldin A at 10 $\mu$g/mL for 6 hours at 37°C. As being intra cellularly located, cytokine expression may be assessed by intracellular flow cytometry. Intracellular flow cytometry typically involves the permeabilization and fixation of the cells. Any convenient means of permeabilizing and fixing the cells may be used in practicing the methods. For example permeabilizing agent typically include saponin, methanol, Tween® 20, Triton X-100TM

Typically, the predetermined reference value is a threshold value or a cut-off value. Typically, a "threshold value" or "cut-off value" can be determined experimentally, empirically, or theoretically. A threshold value can also be arbitrarily selected based upon the existing experimental and/or clinical conditions, as would be recognized by a person of ordinary skilled in the art. For example, retrospective measurement in properly banked historical subject samples may be used in establishing the predetermined reference value. The threshold value has to be determined in order to obtain the optimal sensitivity and specificity according to the function of the test and the benefit/risk balance (clinical consequences of false positive and false negative). Typically, the optimal sensitivity and specificity (and so the threshold value) can be determined using a Receiver Operating Characteristic (ROC) curve based on experimental data. For example, after determining the quantification of the selected population in a group of reference, one can use algorithmic analysis for the statistic treatment of the expression levels determined in samples to be tested, and thus obtain a classification standard having significance for sample classification. The full name of ROC curve is receiver operator characteristic curve, which is also known as receiver operation characteristic curve. It is mainly used for clinical biochemical diagnostic tests. ROC curve is a comprehensive indicator that reflects the continuous variables of true positive rate (sensitivity) and false positive rate (1-specificity). It reveals the relationship between sensitivity and specificity with the image composition method. A series of different cut-off values (thresholds or critical values, boundary values between normal and abnormal results of diagnostic test) are set as continuous variables to calculate a series of sensitivity and specificity values. Then sensitivity is used as the vertical coordinate and specificity is used as the horizontal coordinate to draw a curve. The higher the area under the curve (AUC), the higher the accuracy of diagnosis. On the ROC curve, the point closest to the far upper left of the coordinate diagram is a critical point having both high sensitivity and high specificity values. The AUC value of the ROC curve is between 1.0 and 0.5. When AUC>0.5, the diagnostic result gets better and better as AUC approaches 1. When AUC is between 0.5 and 0.7, the accuracy is low. When AUC is between 0.7 and 0.9, the accuracy is moderate. When AUC is higher than 0.9, the accuracy is high. This algorithmic method is preferably done with a computer. Existing software or systems in the art may be used for the drawing of the ROC curve, such as: MedCalc 9.2.0.1 medical statistical software, SPSS 9.0, ROCPOWER.SAS, DESIGNROC.FOR, MULTIREADER POWER.SAS, CREATE-ROC.SAS, GB STAT VI0.0 (Dynamic Microsystems, Inc. Silver Spring, Md., USA), etc.

In particular, when more than 1 population of cells are quantified, method of the disclosure comprises calculating a composite score that integrates the weight for each population in the contribution in the risk and comparing the patient's composite score to one or more predetermined reference values for determining whether the patient has or is at risk of having T1D. In particular, the method of the disclosure comprises calculating a composite score that integrates the weight for the following populations: MAIT CCR6+, MAIT CD56+, MAIT CCR6+ CD56+, MAIT CCR6- CD56-, MAIT CD25+ PD1+ and MAIT CD25+ CD56- PD1+.

A further object of the disclosure relates to a kit comprising means for performing the method of the disclosure. Typically, the kit comprises means for quantifying the population of cells. In particular, said means are antibodies as described above. In particular, these antibodies are labelled as described above. Typically, the kits described above will also comprise one or more other containers, containing for example, wash reagents, and/or other reagents capable of quantitatively detecting the presence of bound antibodies. The kit also contains agents suitable for performing intracellular flow cytometry such as agents for permeabilization and fixation of cells. Typically compartmentalised kit includes any kit in which reagents are contained in separate containers, and may include small glass containers, plastic containers or strips of plastic or paper. Such containers may allow the efficient transfer of reagents from one compartment to another compartment whilst avoiding cross-contamination of the samples and reagents, and the addition of agents or solutions of each container from one compartment to another in a quantitative fashion. Such kits may also include a container which will accept the blood sample, a container which contains the antibody(s) used in the assay, containers which contain wash reagents (such as phosphate buffered saline, Tris-buffers, and like), and containers which contain the detection reagent.

The disclosure will be further illustrated by the following figures and examples. However, these examples and figures should not be interpreted in any way as limiting the scope of the disclosure.

**FIGURES:**

Figure 1: **frequencies of MAIT cells in the peripheral blood of control children, T1D recent onset children, control adults and T1D adult patients.** Patients correspond to the Table 1.***p<0.001 Mann-Whitney test.

Figure 2: **frequencies of MAIT CCR6+, MAIT CD25+ and MAIT C56+, cells in the peripheral blood of control children, T1D recent onset children, control adults and T1D adult patients.** Patients correspond to the Table 1. * p<0.05, **p<0.01, ***p<0.001 Mann-Whitney test.

Figure 3: **frequencies of iNKT CCR6+ and iNKT CD25+ cells in the peripheral blood of control children, T1D recent onset children, control adults and T1D adult patients.** Patients correspond to the Table 1. * p<0.05, **p<0.01.

Figure 4: **frequencies of MAIT PD1+ and iNKT PD1+ cells in the peripheral blood of control children and T1D recent onset children.** Patients correspond to the Table 1. * p<0.05.

Figure 5: **frequencies of MAIT BCL2+ and MAIT KLRG1+ cells in the peripheral blood of control children and T1D recent onset children.** Patients correspond to the Table 1. * p<0.05, **p<0.01.

Figure 6: **frequencies of MAIT IFNg+ and iNKT IFNg+ cells in the peripheral blood of control children, T1D recent onset children.** Patients correspond to the Table 1. * p<0.05, **p<0.01.

Figure 7: **frequencies of MAIT TNFa+, MAIT IL4+, MAIT GZB+, and MAIT IL17+ cells in the peripheral blood of control children and T1D recent onset children.** Patients correspond to the Table 1. * p<0.05, **p<0.01, ***p<0.001 Mann-Whitney test.

Figure 8: **principal component analysis of cytometric parameters related to MAIT and iNKT cells markers from peripheral blood of control children, T1D recent onset children and T1D children.**

Figure 9: **ROC curve for the logistic regression model based on variables MAIT CCR6, MAIT CD56, MAIT CCR6 + CD56 +, MAIT CCR6- CD56-, MAIT CD25 + PD1 + and MAIT CD25 + CD56- PD1 +.**

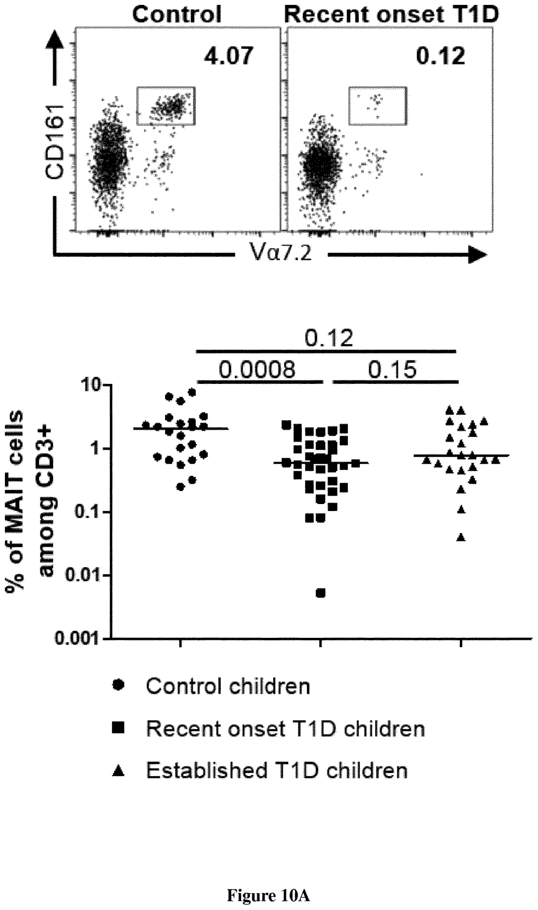

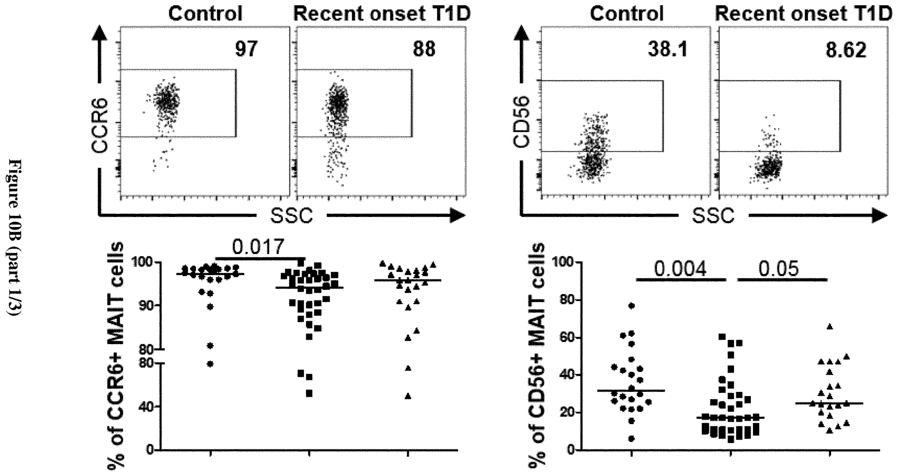

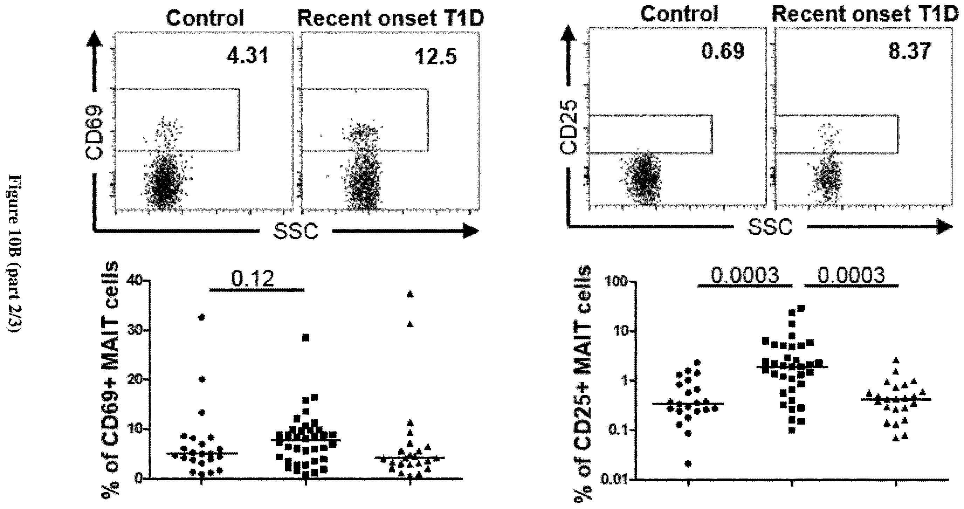

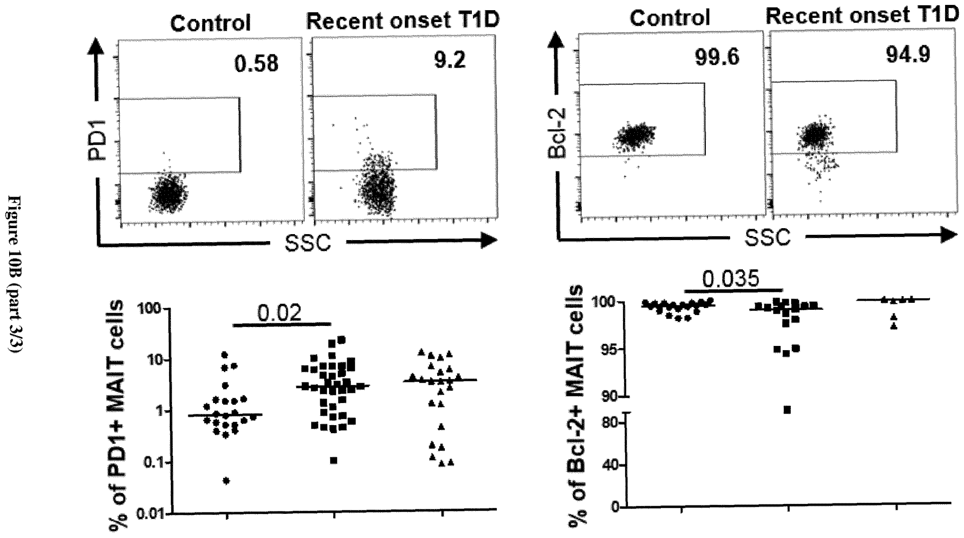

Figure 10: **Frequency and phenotype alterations of blood MAIT cells from T1D children.** MAIT cells were analyzed in blood from children with recent onset T1D (n=41), children with established T1D (n=23), as compared with control children (n=22). (a) Representative staining of MAIT cells and MAIT cell frequency among T lymphocytes. (b) Representative staining and frequency of MAIT cells expressing different cell surface molecules (CCR6, CD56, CD69, CD25, PD1) and intracellular Bcl-2. P values were determined by Kruskal-Wallis test followed by the Wilcoxon rank sum test adjusted with the Holm method.

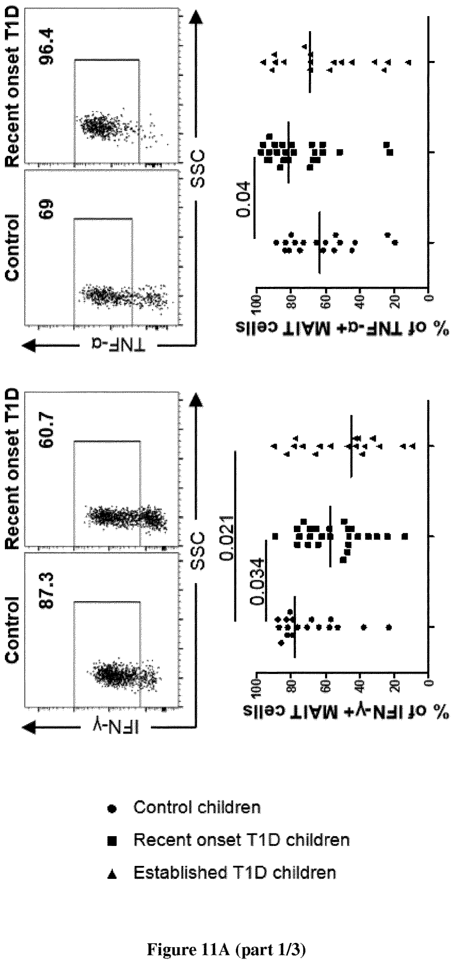

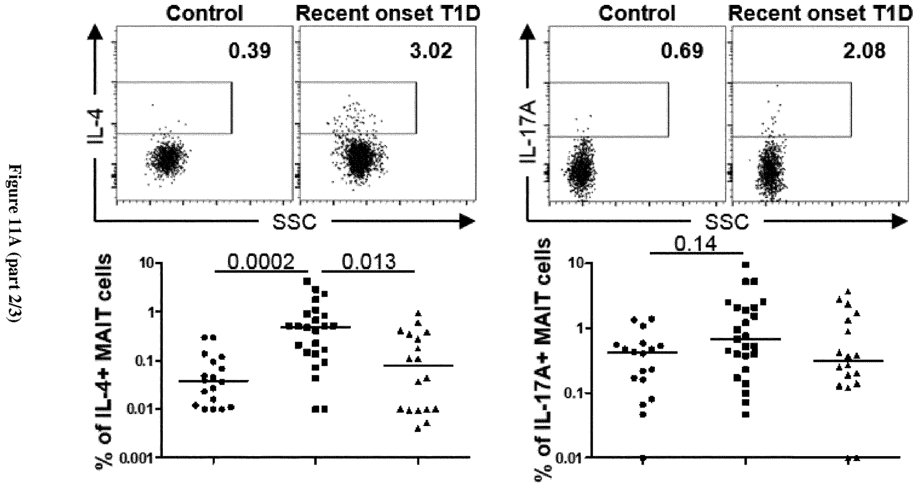

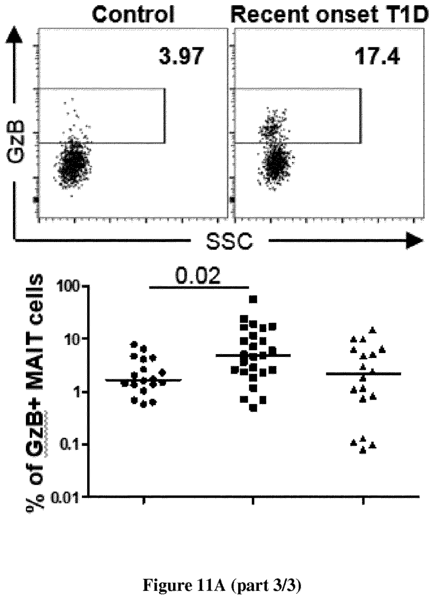

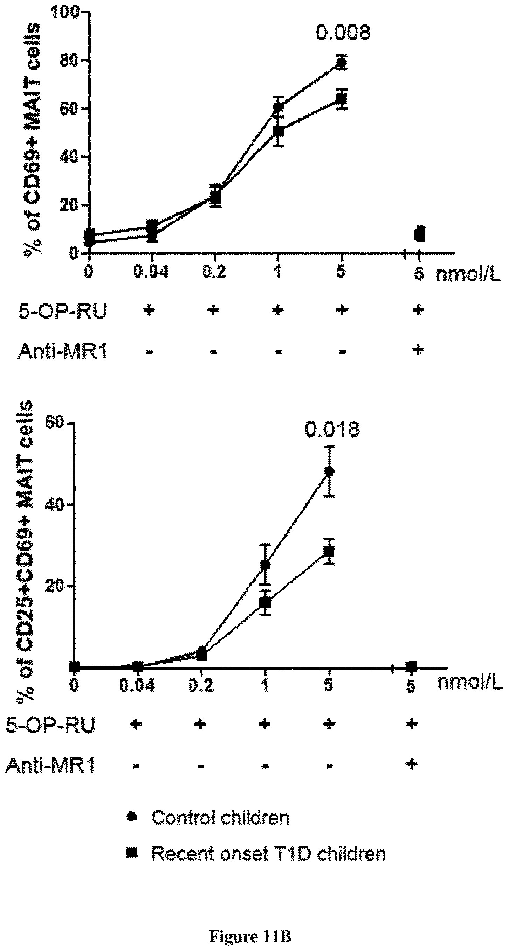

Figure 11: **Functional alterations of blood MAIT cells from T1D children.** MAIT cells were analyzed in blood from children with recent onset T1D (n=25) and children with established T1D (n=18), as compared with control children (n=18). (a) Representative intracellular staining of MAIT cells for cytokines and GzB after PMA/ionomycin stimulation and graphs showing the frequency of MAIT cells producing cytokines and GzB. P values were determined by Kruskal-Wallis test followed by the Wilcoxon rank sum test adjusted with the Holm method. (**b**) Frequencies of CD69$^+$ and CD25$^+$CD69$^+$ MAIT cells from children controls (n=6) and children with recent onset T1D (n=7) after ON stimulation with 5-OP-RU at various concentrations (0 to 5 nmol/L). Blocking MR1 mAb was added when indicated. P values were determined by Mann-Whitney test.

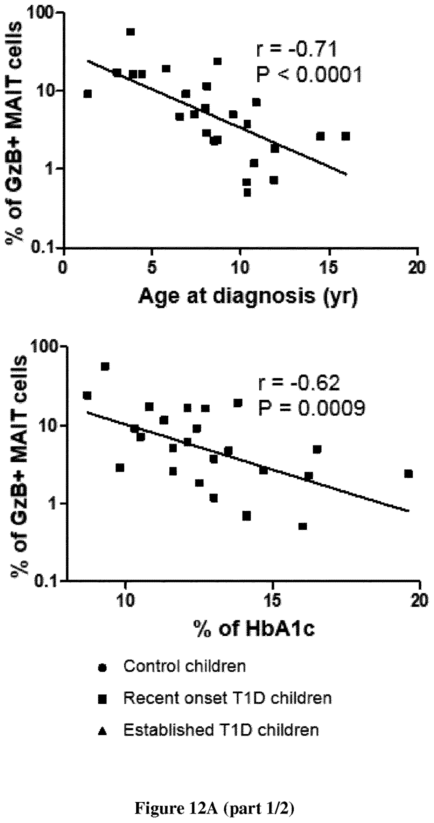

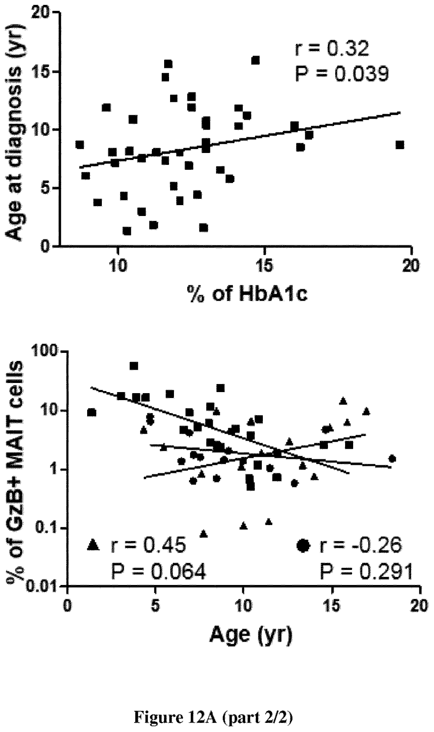

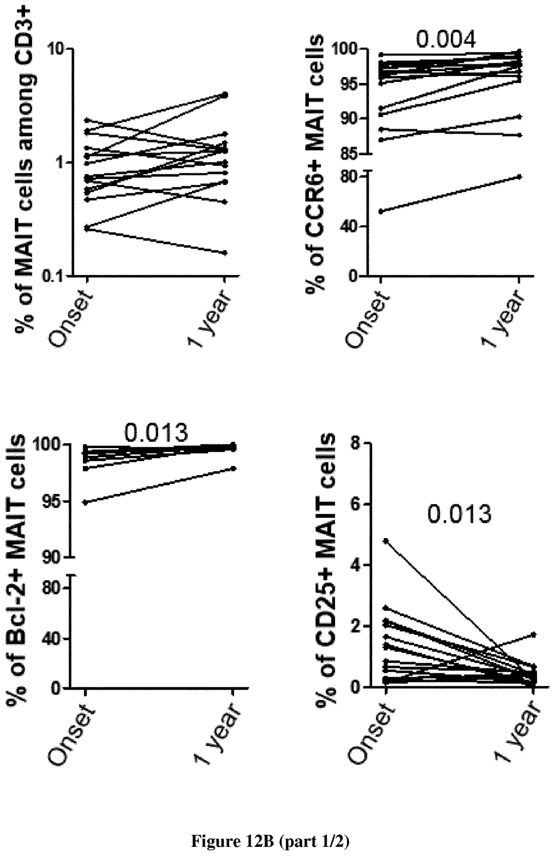

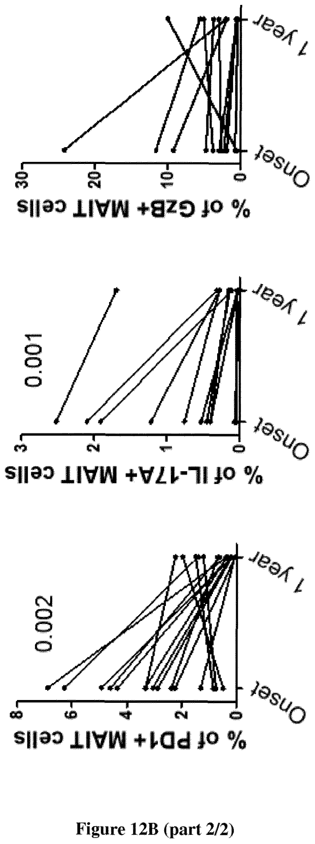

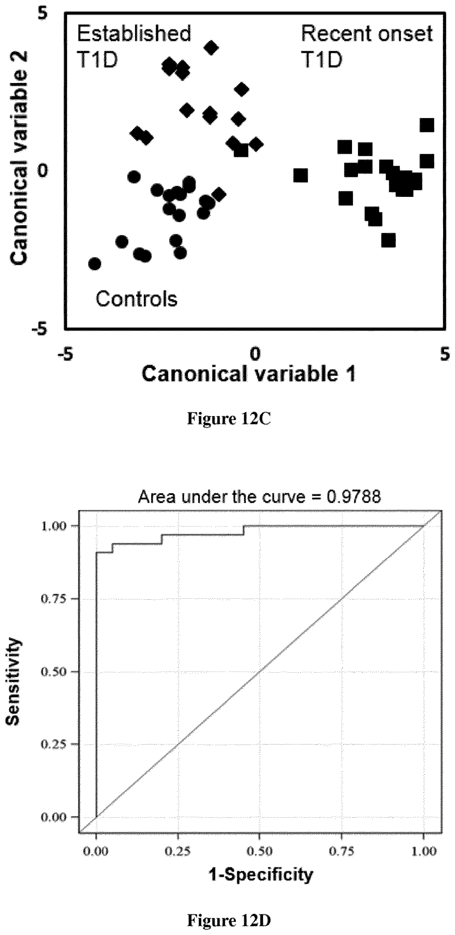

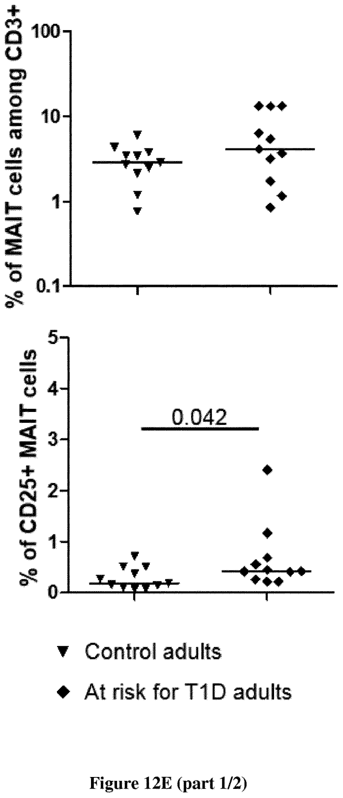

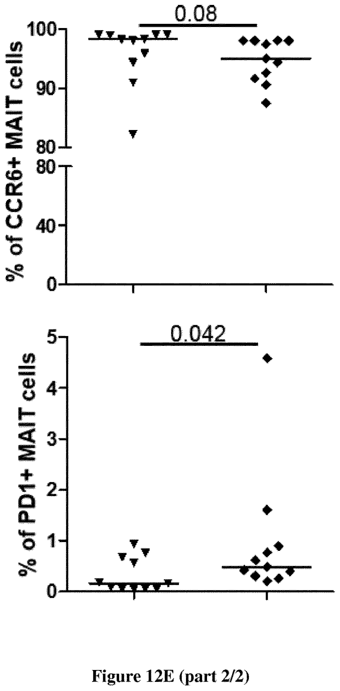

Figure 12: **Relationship between MAIT cell characteristics and disease status of patients,** (**a**) Correlations between the age of children with recent onset T1D (n=25) at diagnosis, the frequency of MAIT cells expressing GzB after PMA/ionomycin stimulation and the HbA1c levels. Correlations between the age and the frequency of MAIT expressing GzB are also shown for the children with established T1D and control children. P value was determined by Spearman test. (**b**) MAIT cell staining of surface and intracellular molecules performed on PBMC from fifteen children with recent onset T1D at the time of diagnosis (on the left) and around one year after diabetes onset (on the right). P values were determined by Signed-rank Wilcoxon Test. (**c**) Factorial discriminant analysis based on the expression of surface and intracellular molecules by MAIT cells from the children with recent onset T1D (n=20), the children with established T1D (n=15) and the children controls (n=18). (**d**) ROC Curve of the predictive model defining the T1D phenotype. (**e**) FACS Analysis of MAIT cell frequency among T lymphocytes and frequency of MAIT cells expressing different cell surface molecules from frozen PBMC of adult controls (n=11) and adult at risk T1D donors with at least two autoantibodies (n=11). P values were determined by Mann-Whitney test for b and e.

**EXAMPLE 1:**

We have observed different alterations of iNKT and MAIT cells quantity, frequency and markers in T1D patients compared to controls and it has also been shown that children with recent onset T1D exhibit different alterations of iNKT and MAIT cells compared to control children or children with established T1D. The alterations should suitable of assessing status, risk or prognosis of type 1 diabetes.

Early alterations of these cell populations would suggest their implication in defective immunoregulation occurring at the pre-diabetic stage. As suggested by our data in mice models (NOD mice), both cell populations could represent new opportunities to avoid deleterious immune response against beta cells and develop strategies to prevent diabetes development. Table 1 describes our current cohort. For the analyses presented below we have reanalyzed all data at the same time to ensure that all samples were studied using the same gating strategy, for increased consistency and precision.

**Table 1 describes the current cohort of patients. Values in parenthesis are range.**

|  | Control Children | Children with recent onset T1D | Control Adults | Adults with long-standing T1D |
|---|---|---|---|---|
| n | 22 | 41 | 29 | 41 |
| Age (Yr) | 9.05 (4.3-18.4) | 8.3 (1.6-16) | 34.9 (23.3-67.3) | 38.9 (17.6-71.1) |
| HbA1c (%) |  | 12.1 (8.7-16.5) |  | 8.07 (5.5-13.5) |
| Duration of disease |  | 4 days (1 - 10) |  | 16.2 years (2-53) |
| BMI / Z-score | -0.3 (-2.2+2.4) | -0.6 (-2.7+3.7) | 21.8 (18.0-27.7) | 23.3 (17.5-37.2) |
| Sex M/F | 13M/9F | 21M/20F | 12M/17F | 15M/26F |

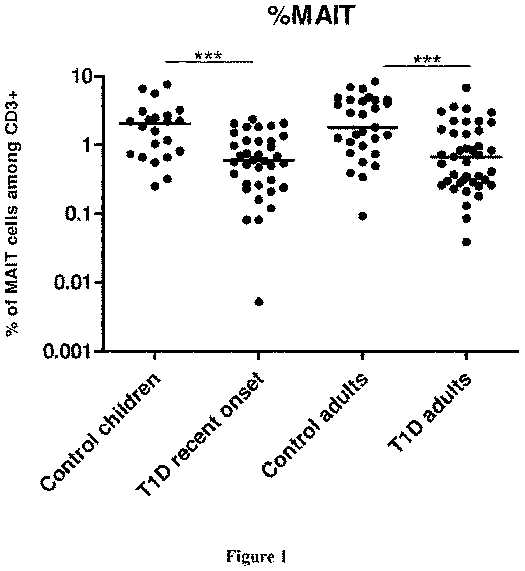

Our results reveal decreased frequencies of iNKT and MAIT cells in children with recent onset T1D (Figure 1). A reduction of MAIT cells was also seen in adult patients with long-standing T1D compared to adult controls (Figure 1).

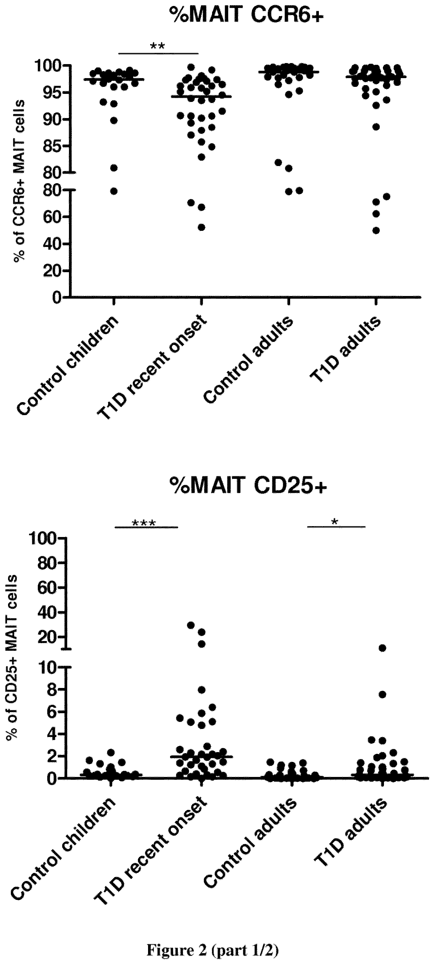

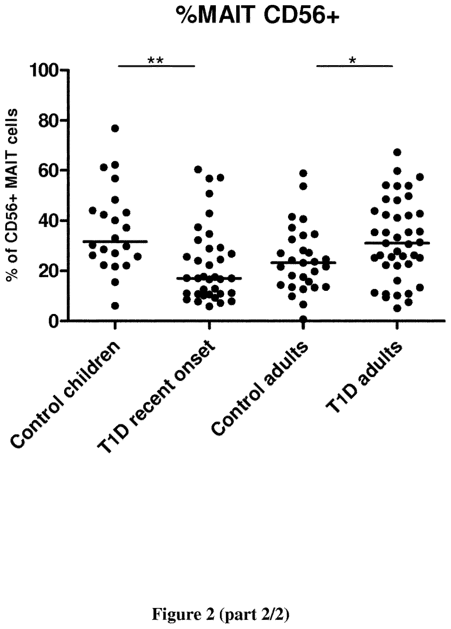

Importantly, a more in depth analysis of the iNKT or MAIT biological markers showed they can be used for the discrimination between controls and patients with recent onset and patient with long-standing T1D. For example, as shown in Figure 2, the quantification of MAIT CCR6+, MAIT CD25+ or MAIT CD56+ cells can be used for such a discrimination. MAIT cells were more often CD25-positive in patient with T1D, when compared to controls. MAIT cells were more often CCR6-negative in patient with T1D recent onset, compared to controls or adults with long-standing T1D. At last MAIT cells were more often CD56-negative in patient with T1D recent onset and can be used to discriminate it from both controls and adults with long-standing T1D (Figure 2).

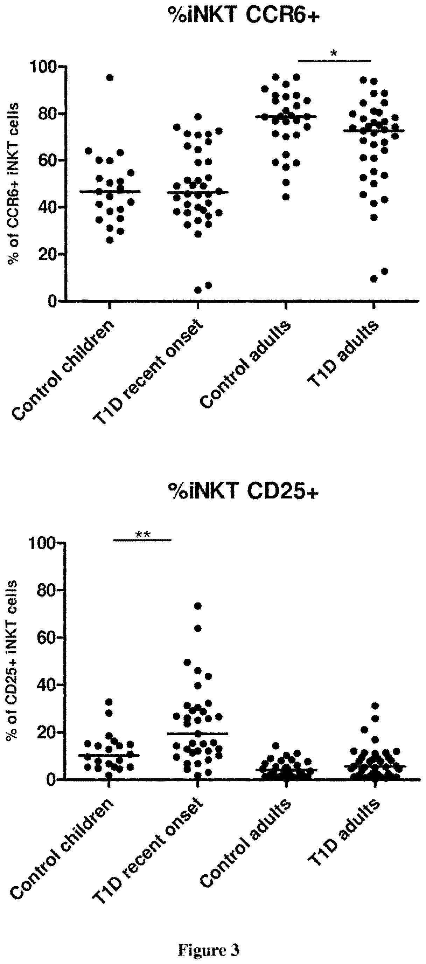

In the same way, iNKT cells were more often CD25-positive in children with recent onset T1D, compared to control children and those with established T1D (Figures 3) whereas iNKT cells were more often CCR6-negative in adults patients with long-standing T1D compared to controls or recent onset (Figures 3). A negative correlation between % CD25+ iNKT cells and iNKT cell frequency is also observed in the adult long-standing T1D group (r=-0.50, p=0.002 **) and in the children recent onset T1D group (r=-0.35, p=0.04*).

For all of these comparisons, the groups were well matched for age. In short, the most striking finding is a decreased iNKT and MAIT cell frequency in the peripheral blood of recent onset T1D children in association with increased proportions of iNKT and MAIT cells expressing the activation marker CD25. Results have been illustrated in Figures 1 to 3 and summarized in Table 2. Of note, there is a positive correlation between the % of CD25+ MAIT cells and the % of CD25+ iNKT cells among the two groups of T1D patients.

**Table 2 describes the different quantification of cells in the patients of our cohort.**

| Test Mann-Whitney | | Controls children | Children with T1D Recent Onset | Controls adults | Children with T1D Follow up |
|---|---|---|---|---|---|
| FREQUENCY | %MAIT | +++ | - | +++ | - |
|  | %iNKT | +++ | + | ++ | + |

(continued)

| Test Mann-Whitney | | Controls children | Children with T1D Recent Onset | Controls adults | Children with T1D Follow up |
|---|---|---|---|---|---|
| MIGRATION | MAIT CCR6+ | +++ | + | +++ | ++ |
| | MAIT CD56+ | ++ | - | + | ++ |
| | iNKT CCR6+ | + | + | +++ | ++ |
| | iNKT CD56+ | + | + | + | +/- |
| ACTIVATION | MAIT CD25+ | - | +++ | - | + |
| | iNKT CD25+ | + | +++ | - | +/- |

These data lead us to propose that in T1D patients there is an abnormal activation of both iNKT and MAIT cells. We hypothesize that this could be due to increased levels of proinflammatory cytokines and/or increase levels of iNKT and MAIT cell ligands, which could be in turn linked to gut microbiota dysbiosis. Both cell types recognize bacterial ligands.

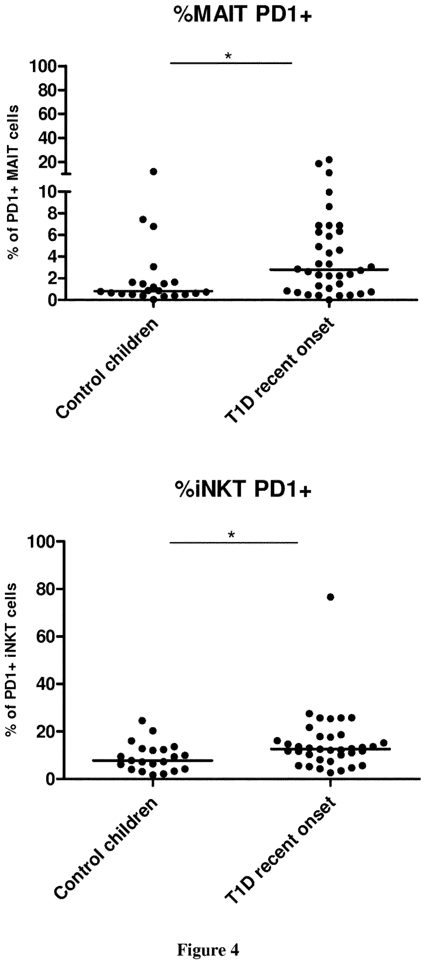

Hence, a specific analysis of differential markers expression on MAIT and iNKT cells from control children compared to recent onset T1D have been conducted. Those expression data for other molecules (PD1, KLRG1, Bcl2...,) have been summarized in Table 3 and some have been illustrated in Figures 4 to 7.

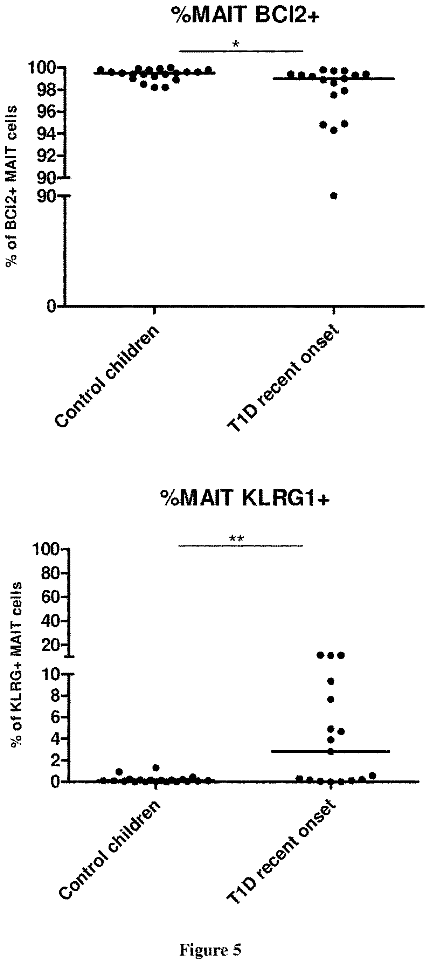

For example, as shown in Figure 4, the quantification of PD1+, which is implicated in cell exhaustion, can be used to discriminate control children from recent T1D onset children. iNKT and MAIT cells were more often PD1-positive in patient with recent T1D, compared to controls (Figure 4). The same can be stated for BCl2+, KLRG1+ (Figure 5).

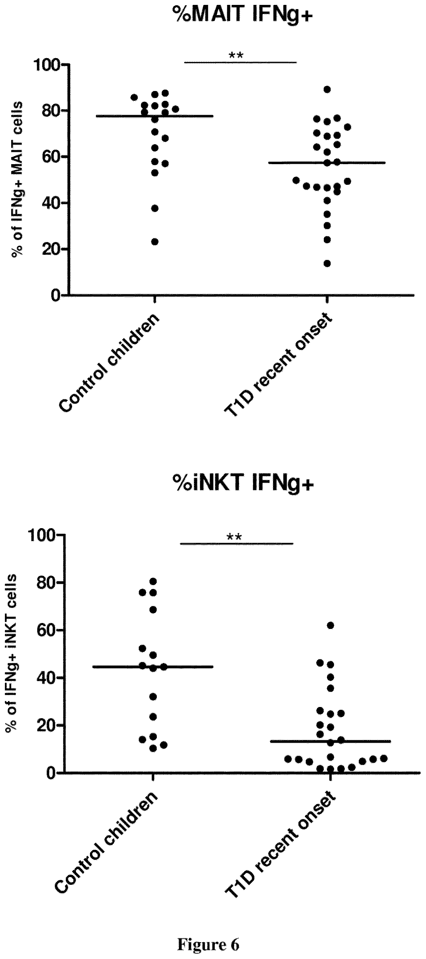

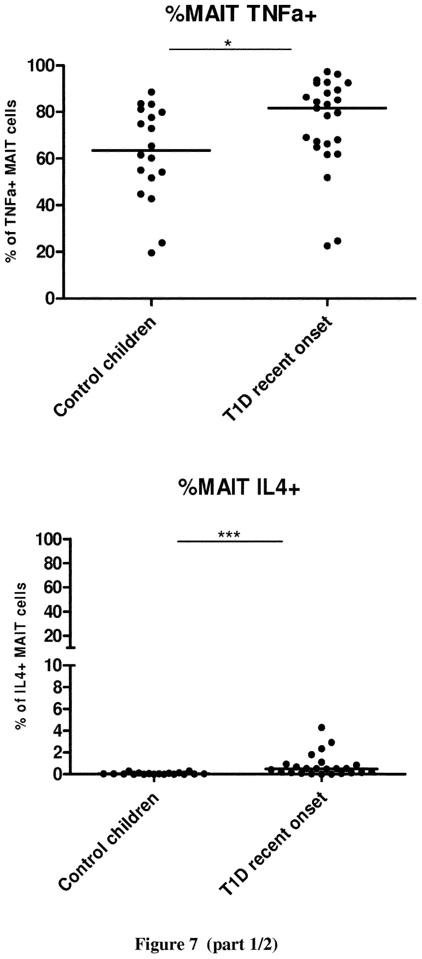

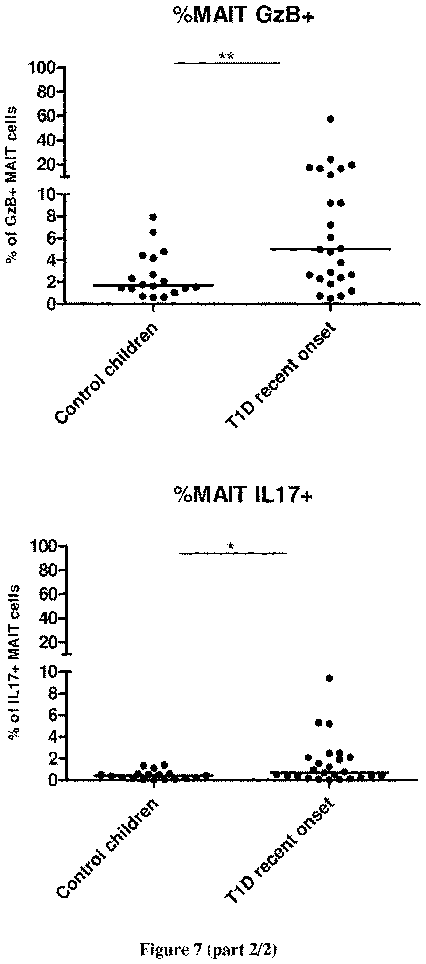

Cytokines and cytotoxic marker GzB, have also been identified, by the inventors, as relevant markers for the discrimination of recent T1D onset as shown in Table 3 and Figures 6 and 7.

**Table 3 describes the different quantification of cells in the patients of our cohort.**

| Test Mann-Whitney | | Controls children | T1D Recent Onset |
|---|---|---|---|
| EXHAUSTION | MAIT PD1+ | - | + |
| | MAIT KLRG1+ | - | + |
| | MAIT BCL2+ | +++ | ++ |
| | iNKT PD1+ | +/- | ++ |
| | iNKT KLRG1+ | - | - |
| | iNKT BCL2+ | +++ | ++ |
| CYTOTOXICITY | MAIT GzB+ | + | +++ |
| | iNKT GzB+ | + | + |

(continued)

| Test Mann-Whitney | | | Controls children | T1D Recent Onset |
|---|---|---|---|---|
| **CYTOKINES** | **Th1** | **MAIT IFNg+** | +++ | ++ |
| | | **MAIT TNFa+** | ++ | +++ |
| | | **iNKT IFNg+** | ++ | - |
| | | **iNKT TNFa+** | ++ | + |
| | **Th2** | **MAIT IL4+** | - | +++ |
| | | **iNKT IL4+** | +/- | +/- |
| | **Th17** | **MAIT IL17+** | - | + |
| | | **iNKT IL17+** | - | - |

Based on those results it can be hypothesized that the decrease of MAIT or iNKT cell frequency observed in the T1D patients and more specifically in the Children with recent onset T1D could be partially explained by their strong activation, leading in turn to their exhaustion.

We also examined the correlation between HbA1c and the proportions of CD25-positive iNKT or MAIT cells in children with recent onset T1D, but there is no correlation. It appears that in this population of patients with very early diagnosis and quite high HbA1c levels, the phenotype of interest does not seem affected by metabolic impairment. The same was observed in children with established T1D and adults with long-standing disease, which tend to have lower HbA1c levels compared to the recent onset patients. Thus, biomarkers identified by the inventors can bring more information on the phenotype of interest compared to HbA1c.

Given these data, the quantification of different population of iNKT and MAIT cells was used to discriminate control children, children with T1D recent onset and children with long standing T1D. The analysis focuses on pediatric population (81 pediatric subjects), itself composed of three sub-populations:

- a population of control, composed of healthy subjects (22 subjects)
- a population of newly diagnosed subjects (41 subjects) and
- a population of diseased subjects (23 subjects).

With this study it was possible to refine the discrimination carried out previously on cytometric parameters and identify biological parameters which allow the best discrimination of the three sub-populations.

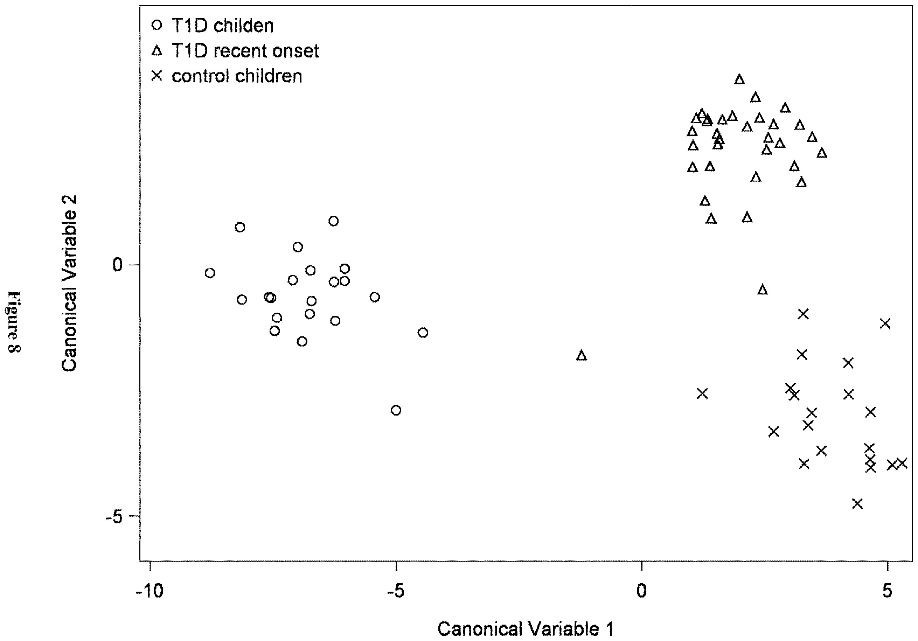

A principal component analysis (PCA) was carried out on the cytometric parameters obtained from MAIT and iNKT cells of those 81 pedriatric subjects (Figure 8). This PCA shows the 86 individuals in the factorial design (abscissa = Can1 and ordered = Can2), with their calculated coordinates. The axis Can1 allows to discriminate one hand sick children (negative values) and secondly controls children and recent diagnosis (positive values). The axis Can2 allows complete discrimination, children newly diagnosed located in the upper right quadrant (positive values, see: Table 5) and the child controls in the lower right quadrant (negative values). Long lasting T1D children are placed themselves between the two groups, close to zero.

Table 4

| Class Means on Canonical Variables | | |
|---|---|---|
| **STATUT** | **Can1** | **Can2** |
| **T1D childen** | -6.768282991 | -0.620392530 |
| **T1D recent onset** | 1.970238152 | 2.224445621 |
| **control children** | 3.855804189 | -3.018923118 |

Can1 and Can2 are based on two new variables also called component, each made as the linear combination of several cytometric parameters. The main factors of Can1 and Can2 are presented in Table 5.

Table 5
**Raw Canonical Coefficients**

| Label | Can1 | Can2 |
|---|---|---|
| **MAIT CD25+** | 140.6887881 | -34.7992235 |
| **MAIT CD25+ PD1+** | -836.668862 | -355.4912666 |
| **MAIT CD25+ PD1-** | -547.1229447 | -195.3184607 |
| **MAIT CCR6+ CD25+ CD56- PD1+** | -380.5641653 | -134.9921849 |
| **iNKT CCR6-CD25+CD161+** | -81.7954022 | -112.6308856 |
| **MAIT CCR6+ CD25+ CD56- PD1-** | -62.8455747 | -59.6811366 |
| **MAIT CCR6- CD25+ PD1-** | -163.9407468 | -51.5567586 |
| **MAIT CCR6- CD25+ CD56- PD1-** | 193.1015985 | -39.0970365 |
| **MAIT CD25+ CD56- PD1+** | 495.9664932 | 72.7252498 |
| **MAIT CCR6+ CD25+ CD56-** | -133.1624776 | 77.4361266 |
| **iNKT CCR6-CD25+CD161+PD1-** | 81.8785878 | 112.9572917 |
| **iNKT CCR6-CD25+CD161+PD1+** | 81.3180599 | 114.1039554 |
| **MAIT CCR6+ CD25+ PD1-** | 467.9625711 | 195.4004921 |
| **MAIT CD25+ CCR6-** | 601.7952535 | 276.8133444 |
| **MAIT CCR6+ CD25+ PD1+** | 764.7454156 | 342.202342 |
| **MAIT CCR6- CD25+ CD56-** | -426.3194369 | 46.5641551 |
| **MAIT CD25+ CD56- PD1-** | 198.1796168 | -16.9507561 |

From Table 5, MAIT and iNKT populations which are highly correlated with recent T1D onset (Can2) or long lasting T1D (Can1) can be identified. It seems that cell populations defined by specific markers combination are more relevant for such discrimination.

Moreover, the analysis of the correlation of each variable with the factor axis, show that the most relevant variables on Can2 (for control vs recent onset discrimination) % MAIT (-22%), iNKT CD25 + (19.1%), MAIT CCR6- PD1 + (19.2%), iNKT CD25 + PD1 + (18.8%).

Such multifactorial analysis, based on several cytometric parameters of MAIT and iNKT cells, allow an efficient discrimination of the three pediatric populations. The representation of individuals in the factorial design (Figure 8) is very relevant. Variable % MAIT (highest correlation with Can2 axis, and p-value = 0.0003), allows to discriminate at best newly diagnosed patients and control patients; healthy individuals tend to have a higher % of MAIT individuals recently diagnosed.

Hence it seems that the study of MAIT or iNKT cells and more particularly the study of CD25, CCR6 or PD1 subpopulation is highly relevant for early diagnosis of T1D.

In order to propose a tool for the risk evaluation or early diagnosis of T1D patient, the inventors have constructed a logistic regression model based of cytometric parameters and status of patients (use of PROC LOGISTIC (SAS 9.3). A method of backward selection was used. Starting from 55 parameters used in the Discriminant Analysis, the selection method backward leads to a model that includes an intercept and variable MAIT CCR6, MAIT CD56, MAIT CCR6 + CD56 +, MAIT CCR6- CD56-, MAIT CD25 + PD1 + and MAIT CD25 + CD56- PD1 +, all significant at 5% (Table 6).

Table 6

| Cytometric parameter | DF | Wald Chi-Square | Pr > ChiSq |
|---|---|---|---|
| MAIT CCR6 | 1 | 5.4091 | 0.0200 |
| MAIT CD56 | 1 | 5.1571 | 0.0232 |
| MAIT CCR6+ CD56+ | 1 | 5.1552 | 0.0232 |
| MAIT CCR6- CD56- | 1 | 5.4440 | 0.0196 |
| MAIT CD25+ PD1+ | 1 | 5.8562 | 0.0155 |
| MAIT CD25+ CD56- PD1+ | 1 | 5.9922 | 0.0144 |

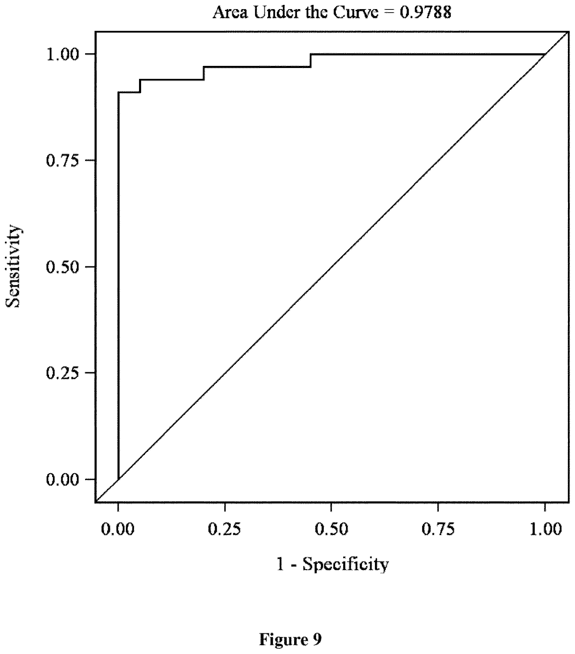

The ROC curve, plotted in Figure 9, assessed the performance of the model to classify subjects based on their status (recent / healthy). The area under the curve (AUC, Area Under Curve) is 0.9788 or very close to the maximum value of 1. This curve shows the sensitivity (proportion of diagnoses classified diagnosed ordinate) versus 1 - specificity (proportion of control subjects classified diagnosed).

The model confusion matrix (for a median threshold s = 0.5) is presented in Table 7. 4 of 53 subjects (7.5%) were misclassified: 2 and 2 control subjects newly diagnosed subjects.

Table 7

| STATUT | Predicted Statut | | |
|---|---|---|---|
| *Frequency Row Pct* | **T1D recent onset** | **control children** | **Total** |
| **T1D recent onset** | 31<br>93.94 | 2<br>6.06 | 33 |
| **control children** | 2<br>10.00 | 18<br>90.00 | 20 |
| **Total** | 33 | 20 | 53 |
| *Frequency Missing = 10* | | | |

Furthermore, from the model coefficients (Table 8), it is possible to diagnose a new patient, according to this data.

**Table 8**

| Variable | Coefficient | Value |
|---|---|---|
| **Intercept** | $\alpha$ | -11040.61 |
| **MAIT CCR6** | $\beta_1$ | 110.368 |
| **MAIT CD56** | $\beta_2$ | 105.802 |
| **MAIT CCR6+ CD56+** | $\beta_3$ | -105.708 |
| **MAIT CCR6- CD56-** | $\beta_4$ | 111.147 |
| **MAIT CD25+ PD1+** | $\beta_5$ | -96.8976 |
| **MAIT CD25+ CD56- PD1+** | $\beta_6$ | 154.676 |

Such diagnosis can be made using the following logistic regression based formula, with $\pi$ the probability of a subject to be diagnosed sick:

$$logit(\pi) = \log\left(\frac{\pi}{1-\pi}\right)$$
$$= \alpha + \beta_1 \times MAIT\ CCR6 + \beta_2 \times MAIT\ CD56 + \beta_3 \times MAIT\ CCR6_+ CD56_+$$
$$+ \beta_4 \times MAIT\ CCR6_- CD56_- + \beta_5 \times MAIT\ CD25_+ PD1_+$$
$$+ \beta_6 \times MAIT\ CD25_+ CD56_- PD1_+$$

Noting X'β the previous result

$$\pi = \frac{\exp(X'\beta)}{1 + exp(X'\beta)}$$

For example, two hypothetical patients were defined (Table 9). Given their respective values for the values of the model, the patient would be diagnosed patient A ($\pi \geq 0.6$), and the patient would be diagnosed healthy B ($\pi <0.6$).

Table 9

| Sujet | Variable | Valeur | X'β | π |
|---|---|---|---|---|
| Patient A | MAIT CCR6 | 95 | 1.042 | 0.73932 |
| | MAIT CD56 | 10 | | |
| | MAIT CCR6+ CD56+ | 10 | | |
| | MAIT CCR6- CD56- | 5 | | |
| | MAIT CD25+ PD1+ | 0 | | |
| | MAIT CD25+ CD56- PD1+ | 0 | | |
| Patient B | MAIT CCR6 | 95 | -331.459 | <0.0001 |
| | MAIT CD56 | 20 | | |
| | MAIT CCR6+ CD56+ | 20 | | |
| | MAIT CCR6- CD56- | 2 | | |
| | MAIT CD25+ PD1+ | 0 | | |
| | MAIT CD25+ CD56- PD1+ | 0 | | |

Such model have also been used on a data set which has not been used for the regression model construction (Table 10).

Table 10

| Statut | Variables | Values | X'β | π |
|---|---|---|---|---|
| T1D recent onset | MAIT CCR6+ | 97.7 | 8.8087808 | 0.99985061 |
| | MAIT CD56+ | 17.7 | | |
| | MAIT CCR6+ CD56+ | 17.5 | | |
| | MAIT CCR6- CD56- | 2.16 | | |
| | MAIT CD25+ PD1+ | 0.062 | | |
| | MAIT CD25+ CD56- PD1+ | 0.062 | | |
| T1D recent onset | MAIT CCR6+ | 94.5 | 367.497568 | 1 |
| | MAIT CD56+ | 11 | | |
| | MAIT CCR6+ CD56+ | 11 | | |
| | MAIT CCR6- CD56- | 5.48 | | |
| | MAIT CD25+ PD1+ | 8.22 | | |
| | MAIT CD25+ CD56- PD1+ | 7.53 | | |
| control | MAIT CCR6+ | 98.6 | -13.94301 | 8.8029E-07 |
| | MAIT CD56+ | 40.1 | | |
| | MAIT CCR6+ CD56+ | 40 | | |
| | MAIT CCR6- CD56- | 1.17 | | |
| | MAIT CD25+ PD1+ | 0 | | |
| | MAIT CD25+ CD56- PD1+ | 0 | | |
| control | MAIT CCR6+ | 92.9 | -2.9174316 | 0.05129855 |
| | MAIT CD56+ | 33 | | |
| | MAIT CCR6+ CD56+ | 32.2 | | |
| | MAIT CCR6- CD56- | 6.23 | | |
| | MAIT CD25+ PD1+ | 0.076 | | |
| | MAIT CD25+ CD56- PD1+ | 0.076 | | |

Such results confirmed the effectiveness of the quantification of MAIT or iNKT cells and more particularly their subpopulation for the follow up of T1D pathology and more particularly it early diagnosis.

To conclude, dysregulation of iNKT and MAIT cells may occur time before diabetes diagnosis, during the prediabetes period and might modify the balance between the activating and inhibiting ligands of MAIT and iNKT cells in such a way that both cell types could be activated before the multiple seroconversion state.

**EXAMPLE 2:**

**Material and methods**

**Human samples.** Peripheral blood samples were obtained from control children and from T1D children admitted in the Pediatric Endocrinology department of Necker hospital, Paris, France at T1D onset (i.e. within 10 days from first insulin injection), or with established disease. None of the control children had a personal or familial history of T1D or autoantibodies associated with T1D. Non-inclusion clinical following parameters contain: infection during the admission and associated others autoimmune disease. The Ethics Committee (Comité de protection des personnes (CPP) Ile-de-France) approved the clinical investigations and written informed consent was obtained from all the parents. For the Milan cohort, peripheral blood from healthy control subjects and patients at T1D onset (i.e., within 10 days from first insulin injection) were collected. The study was approved by the San Raffaele Hospital Ethic Committee (protocol: DRI-003). At risk subjects were enrolled in the Type 1 Diabetes TrialNet Pathway to Prevention Trial (TN01 trial, former TrialNet Natural History Study). The overall objective of this study is to perform baseline and repeated assessments over time of the immunologic and metabolic status of individuals who are at risk for T1D (first/second degree relatives of patients with T1D). The study was approved by the San Raffaele Hospital Ethics Committee (protocol: NHPROTOCOL32803 TN01). Our local study was approved by the TrialNet Ancillary Studies Subcommittee. All analyses were performed blinded and all subjects included in this study signed the informed consent prior to blood donation.

**Cell preparations.** Human peripheral blood mono-nucleated cells (PBMC) of patients from Necker Hospital were isolated from fresh blood samples by Ficoll-Paque (Leucosep) or samples from San Raffaele hospital were defrosted in RPMI with 10% FCS (Fetal Cow Serum).

**Flow cytometry and antibodies.** Cells were stained in PBS containing 5% FCS and 0.1% azide. For human PBMC the following antibodies were used: CD3 (OKT3), CD4 (OKT4), V$\alpha$7.2 (3C10), CD161 (HP-3G10), CCR6 (G034E3), CD56 (HCD56), CD69 (FN50), Bcl-2 (100), IFN-$\gamma$ (4S B3), IL17A (BL168), TNF-$\alpha$ (MAb11) mAbs from Bio-Legend; CD8 (SK1), PD1 (MIH4), CD25 (M-A251), IL4 (8D4-8), granzyme B (GB11) mAbs from BD Biosciences; CD3 (REA), CD161 (REA) mAbs from Miltenyi and CD4 (RPA-T4) mAb from eBiosciences. Data acquisition was performed using BD Biosciences LSRFortessa cytometer or FACS ARIA III cytometer for cells from patients from Necker hospital and Beckman Coulter Gallios for cells from patients from San Raffaele hospital.

**In vitro cell stimulation.** For ligand stimulation of human MAIT cells, 5-OP-RU solution was obtained after incubating 1 molar equivalent of 5-ARU with 2 molar equivalent of methylglyoxal (Sigma-Aldrich). Hela cells and PBMC from children controls and children with recent onset T1D were plated at a final concentration of 500 000 cells/mL each on 24-well plate, in RPMI supplemented with 10% FCS from children controls and children with recent onset T1D were plated at a final concentration of 106 cells/mL in RPMI supplemented with 10% FCS. PBMC were stimulated ON with various concentration of 5-OP-RU (0-5 nmol/L). Blocking MR1 (26.5 mAb) from Biolegend was added when indicated at 10 $\mu$g/mL. MAIT cell activation was analyzed by flow cytometry.

**Intra-cellular staining.** For human Bcl-2 staining, after surface staining lymphocytes were resuspended in fixation/permeabilization buffer (Foxp3 staining kit from eBioscience) and incubated at 4°C in the dark then, cells were washed with PERM Wash buffer (eBioscience) and labeled with appropriate mAbs. For cytokine and granzyme B analysis of human MAIT cells, PBMC obtained from fresh samples, were analyzed after stimulation for 6 h at 37°C in RPMI medium supplemented with 10% FCS with PMA (25 ng/ml) and ionomycin (1 $\mu$g/ml), in the presence of Brefeldin A (10 $\mu$g/ml).

**Statistical analysis.** For human studies, statistical tests between two groups were performed using two-tailed Mann-Whitney test and signed-rank Wilcoxon test with Graph Pad Prism. The Kruskal-Wallis test followed by the Wilcoxon rank sum test adjusted with the Holm method and the Spearman correlation test was applied for all the correlation analysis with Software R. A factorial discriminant analysis was performed using the XLSTAT 2016 Software. A logistic regression model was fitted with the PROC CANDISC software (SAS version p.3) then a backward elimination procedure was applied. Prognostic validity of the model was evaluated by the receiver operating characteristic (ROC) curve analysis and measured using the area under the ROC curve (AUC). Statistical analyses were performed using the GraphPad Prism software version 5.00.288 and the R software version 3.2.3.

### Results

**Alteration of blood MAIT cell frequency and phenotype in children with recent onset T1D**

We first began the investigation of MAIT cells in T1D by analyzing MAIT cell frequency and phenotype in fresh peripheral blood samples from children with recent onset T1D and children with established T1D as compared to age-matched control children **(data not shown)**. MAIT cells can be identified in human blood as CD4- T lymphocyte expressing V$\alpha$7.2 TCRa gene segment and CD161high **(Fig. 10a)**. MAIT cell frequency and number was decreased (3-fold) in the blood of recent onset T1D children whereas no significant difference was observed in children with established disease as compared to control children **(Fig. 10a and data not shown)**. Decreased frequency was observed in both CD8+ and double negative (DN) MAIT cell subsets **(data not shown)**. Of note there was no difference in the frequencies of conventional CD4 and CD8 T cells, and of V$\alpha$7.2+CD161- T cells between the three children populations confirming that the decrease of MAIT cell frequency at the onset of T1D was not consecutive of changes in other T cell populations nor to down-regulation of the CD 161 marker **(data not shown)**. Analysis of MAIT cell phenotype showed a decreased frequency of MAIT cells expressing tissue recruitment/adhesion molecules (CCR6, CD56) at the onset of the disease, an increased frequency of MAIT cells expressing the activation/exhaustion markers CD25 and PD1, and a decreased frequency of MAIT cells expressing the anti-apoptotic molecule Bcl-2 **(Fig. 10b and data not shown)**. Multi-parametric analysis of MAIT cells in the children with established T1D highlighted the intermediate phenotype of MAIT cells between those from recent onset T1D and control children **(data not shown)**. Interestingly in recent onset children the frequency of MAIT cells expressing migratory CCR6+ or anti-apoptotic Bcl-2 molecules were positively correlated with the frequency of MAIT cells, whereas MAIT cell CD25 expression was negatively correlated with MAIT cell frequency **(data not shown)**. These data suggest that decreased blood MAIT cell frequency could reflect their migration to inflamed tissues and/or their death by apoptosis subsequent to their activation.

**Alteration of blood MAIT cell function in children with recent onset T1D**

Cytokine and GzB production by fresh blood MAIT cells was analyzed after PMA-ionomycin stimulation. MAIT cells from children with recent onset T1D produced less IFN-y, whereas their production of TNF-$\alpha$, IL-4, and GzB was increased as compared with MAIT cells from control children **(Fig. 11a and data not shown)**. Of note, among these effector molecules only the frequency of GzB correlated with the frequency of MAIT cells, the higher GzB production was observed in patients with the lower frequency of MAIT cells **(data not shown)**. Multi-parametric analysis of cytokines and GzB production by MAIT cells also showed an intermediate status of blood MAIT cells from children with established T1D, between those from control children and recent onset T1D children, as already observed for MAIT cell surface phenotype **(data not shown)**. We next analyzed the ability of MAIT cells to respond to specific TCR activation by the ligand 5-OP-RU. Upon stimulation, MAIT cells from control children up-regulated CD69 and CD25 activation markers. Addition of blocking MR1 mAb confirms that this activation was TCR-dependent. Interestingly, MAIT cell activation was significantly reduced in children with recent onset T1D **(Fig. 11b)**. Together, these results highlight functional alteration of MAIT cells in children with recent onset T1D.

**Association between MAIT cell alterations and clinical characteristics**

We next investigated potential links between phenotype and functional alterations of MAIT cells and clinical characteristics of children with recent onset T1D **(data not shown)**. Interestingly, the frequency of GzB+ MAIT cells was negatively associated (r=-0.71, P<0.0001) with children's age at diagnosis **(Fig. 12a),** which is in agreement with the current view that T1D is more aggressive in the youngest children. We speculate that production of GzB by MAIT cells, reflecting their cytotoxic potential, could be involved in the physiopathology of T1D. Other MAIT cell parameters, such as their frequency, CCR6 and Bcl-2 expression, were also associated with the age at diagnosis **(data not shown)**. No significant correlations between MAIT cell parameters and age were observed in controls and children with established T1D (data not shown). Production of GzB by MAIT cells inversely correlated with HbA1c level at the onset of the disease but not in children with established T1D **(Fig. 12a)**. Indeed, a more aggressive disease associated with sustained MAIT cell abnormalities suggests a shorter time of hyperglycemia before the onset thereby lower levels of HbA1c.
To further explore the link between MAIT cell parameters and clinical characteristics, 15 of the children with recent onset T1D were further analyzed one year later. Although MAIT cell frequency among T cells remain to a similar level after one year of insulin, both CCR6+ and Bcl-2+ MAIT cell frequencies significantly increased. Conversely the frequencies of CD25+, PD1+, IL-17A+, and to some extent GzB+, MAIT cells decreased to levels observed in the control children **(Fig. 12b)**. This longitudinal analysis strengthened the data obtained in the transversal analysis showing that MAIT cell phenotype was more similar to controls in the children under insulin therapy than at disease onset.

**MAIT cells as a new biomarker of T1D**

Canonical analysis was performed to compare MAIT cell alterations in the three groups of children (controls, recent onset and established T1D). This analysis revealed that MAIT cell parameters were sufficient to discriminate the three groups of children analyzed **(Fig. 12c).** Moreover a statistical regression analysis identified four surface markers that define a predictive model for the diagnosis of the disease tested on the ROC curve **(Fig. 12d).**

Importantly, we confirmed in another cohort of children from Milano that frequency and phenotype alteration of MAIT cell was observed in recent onset T1D as compared to age-matched control children **(data not shown).** However technical difficulties impacting CD56 analysis on these frozen cells did not allow to applying the predictive model.

Finally, to test whether MAIT cell alterations could be detected before the onset of diagnosis, we characterized MAIT cells in adults at risk to develop T1D defined as direct relatives of T1D patients with at least two positive autoantibodies **(data not shown).** As compared to the control group, there were increased frequencies of $CD25^+$ and $PD1^+$ MAIT cells and a trend toward a lower $CCR6^+$ MAIT cell frequency, even though the number of individuals analyzed were limited **(Fig. 12e).** Altogether our data in patients suggest that MAIT cells represent a new biomarker in T1D and they could play a role in the pathogenesis of T1D. Therefore we investigated MAIT cells in mouse models, which allow analysis in tissues at different stages of disease development and could be manipulated to determine whether MAIT cells are involved in T1D physiopathology.

**REFERENCES:**

Throughout this application, various references describe the state of the art to which this disclosure pertains.

**Claims**

1. A method of identifying whether a subject has or is at risk of having type 1 diabetes comprising

   - i) quantifying the population of MAIT cells (Mucosal-Associated Invariant T cells) in the blood sample obtained from the subject, ii) comparing the quantification value determined at step i) with a predetermined reference value and iii) concluding that the subject has or is at risk of having type 1 diabetes when the quantification value at step i) is lower than the predetermined reference value.

2. The method of claim 1 wherein the subject is a child or an adult.

**Patentansprüche**

1. Verfahren zum Identifizieren, ob ein Subjekt Typ-1-Diabetes oder das Risiko dafür aufweist, umfassend

   - i) Quantifizieren der Population von MAIT-Zellen (Mucosa-assoziierte invariante T-Zellen) in der von dem Subjekt gewonnenen Blutprobe, ii) Vergleichen des in Schritt i) bestimmten Quantifizierungswerts mit einem vorbestimmten Referenzwert und iii) Schlussfolgern, dass das Subjekt Typ-1-Diabetes oder das Risiko dafür aufweist, wenn der Quantifizierungswert in Schritt i) niedriger als der vorbestimmte Referenzwert ist.

2. Verfahren nach Anspruch 1, wobei das Subjekt ein Kind oder ein Erwachsener ist.

**Revendications**

1. Procédé pour identifier si un sujet présente ou risque de présenter un diabète de type 1 comprenant

   - i) la quantification de la population de lymphocytes MAIT (lymphocytes T invariants associés aux muqueuses) dans l'échantillon de sang obtenu du sujet, ii) la comparaison de la valeur de quantification déterminée à l'étape i) avec une valeur de référence prédéterminée et iii) la conclusion établissant que le sujet présente ou risque de présenter un diabète de type 1 lorsque la valeur de quantification à l'étape i) est inférieure à la valeur de référence prédéterminée.

2. Procédé selon la revendication 1, dans lequel le sujet est un enfant ou un adulte.

Figure 1

## %MAIT CCR6+

## %MAIT CD25+

Figure 2 (part 1/2)

Figure 2 (part 2/2)

# %iNKT CCR6+

# %iNKT CD25+

Figure 3

Figure 4

## %MAIT BCl2+

## %MAIT KLRG1+

**Figure 5**

## %MAIT IFNg+

## %iNKT IFNg+

**Figure 6**

## %MAIT TNFa+

## %MAIT IL4+

**Figure 7 (part 1/2)**

Figure 7 (part 2/2)

**Figure 8**

Figure 9

**Figure 10A**

**Figure 10B (part 1/3)**

**Figure 10B (part 2/3)**

**Figure 10B (part 3/3)**

● Control children

■ Recent onset T1D children

▲ Established T1D children

**Figure 11A (part 1/3)**

**Figure 11A (part 2/3)**

35

**Figure 11A (part 3/3)**

Figure 11B

● Control children

■ Recent onset T1D children

▲ Established T1D children

**Figure 12A (part 1/2)**

Figure 12A (part 2/2)

**Figure 12B (part 1/2)**

**Figure 12B (part 2/2)**

Figure 12C

Figure 12D

**Figure 12E (part 1/2)**

**Figure 12E (part 2/2)**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HARMS R.Z et al.** *Plos One,* 2015
- **MAGALHAES I et al.** *Frontiers in Immunology,* 2015
- **KOSTIC et al.** *Cell Host & Microbes,* 2015
- **BOSI E et al.** *Diabetologia,* 2006
- **GRAHAM S et al.** *Gut,* 2004
- **LANTZ ; BENDELAC.** *J. Exp Med.,* 1994, vol. 180, 1097-106
- **TILLOY et al.** *J. Exp. Med.,* 1999, 1907-1921
- **TREINER et al.** *Nature,* 2003, vol. 422, 164-169
- **ROITT, I.** Essential Immunology. Blackwell Scientific Publications, 1991